# EUROPEAN PATENT APPLICATION

(11) **EP 1 415 681 A1**
(43) Date of publication of application: **06.05.2004**
(21) Application number: 02760581.5
(22) Date of filing: 08.08.2002
(51) Int. Cl.: A61M 25/10

(54) **EXPANSION CATHETER**

(30) Priority: 08.08.2001 JP 2001241377; 24.08.2001 JP 2001255396
(71) Applicant: Kaneka Corporation, Osaka-shi, Osaka 530-0005 (JP)
(72) Inventor: FUKAYA, Kohei, Settsu-shi, Osaka 566-0072 (JP)
(74) Representative: HOFFMANN EITLE
(86) International application number: PCT/JP2002/008099
(87) International publication number: WO 2003/013642

(57) **Abstract**

By installing a cutting blade structure onto the tubular members in the vicinity of the expandable body, the expandable body section can be made fine and flexible, and a dilatation catheter of excellent operability can be obtained, particularly in terms of its capacity to be advanced through constricted affected regions or curved affected regions. Moreover, it is also possible freely to control the expandability characteristics (compliance) of the expandable body. Furthermore, by providing protecting members which protect the blade section of the cutting blade, it is possible to increase the protection of the cutting blade, thereby providing a dilatation catheter which has both excellent safety characteristics and improved flexibility in the region of the expandable body.

## Description

### BACKGROUND OF THE INVENTION

### 1. Field of the Invention

The present invention relates to an dilatation catheter used for surgery whose object is a dilatation operation of an internal body passage, and more particularly, it relates to an dilatation catheter which prevents unintentional damage or injury to the walls of the internal body passage, by inserting an incision into the portion that is to be dilated whilst dilating same.

### 2. Description of the Related Art

A dilatation catheter is mainly used in surgery for performing angioplasty in internal body passages, such as constricted or occluded blood vessels. In general, a dilatation catheter has a structure having an expandable body wherein dilatation lumens for supplying a pressurized fluid are coupled to the front end portion of a tube-shaped catheter shaft which comprises a plurality of lumens therein, and ports connecting to the respective lumens in the base portion thereof, and in a normal state, the expandable body is folded over the catheter shaft. In surgery, the expandable body of the dilatation catheter is, for example, inserted via a main artery of the patient and into the constricted area of a coronary artery, where it is caused to expand by introducing a pressurized fluid into the expandable body, thereby dilating the affected part that is constricted or occluded.

However, in this dilatation treatment, there has been a problem in that, at the same time, excessive damage is caused to the tissue of the internal passage, such as the blood vessel. In other words, the dilatation treatment causes the blood vessel, or other internal passage, to expand whilst being torn open by the internal pressure, but in this case, excessive stress and friction is applied to the tissue of the walls of the internal passage. In this case, the excessive damage caused can lead to consequences such as a critical obstruction or thrombosis immediately after dilatation, or reocculsion of the passage due to the growth of additional tissue caused by the scarring, in a chronic fashion.

In order to resolve these problems, Japanese Patent No. 2591573 discloses an dilatation catheter which is constituted by an expandable body, and a plurality of atheroma cutters (metal blades attached to the expandable body by polyurethane adhesive) which are attached to the outer face of the expandable body, with the object of reducing the stress applied to the tissue of the internal body passages, such as the blood vessels, during dilatation treatment.

However, this dilatation catheter consisting of an expandable body and a plurality of atheroma cutters·installed on the outer face of the expandable body involves problems in that it is limited in terms of the swelling properties of the expandable body, due to structural drawbacks thereof, and the flexibility of the expandable body section is poor, thereby leading to poor passage through to the affected area, and the like.

To give a detailed description, since the expandable body comprises atheroma cutters fixed to the surface thereof by means of adhesive, the expandability characteristics (compliance), being the relationship between the pressure and the diameter, must be very small. The reason for this is that if the expandability characteristics (compliance) is high, then the fixing of the atheroma cutters by adhesion will become unstable due to the expansion (deformation) of the surface of the expandable body, as it swells. Consequently, it has been necessary to select an expandable body having low expandability characteristics, and an expandable body having expandability characteristics of this kind is inevitably hard in the thickness direction thereof.

Furthermore, the method of fixing the atheroma cutters directly to the expandable body by means of an adhesive also has a negative effect on the flexibility of the expandable body. Since the blades are always held facing in the outward circumferential direction, sufficient strength is required in order to ensure safety, and the adhesive itself is also required to have a certain degree of hardness, and when a hard expandable body and hard glue of this kind, and a plurality of atheroma cutters, are fixed together as a single unit, the expandable body section of the catheter will have a very large profile and will be extremely hard. On the other hand, in dilatation treatment of internal body passages, in many cases, particularly those relating to blood vessels, it is necessary to insert the catheter along the blood vessel from the insertion opening up to the affected area or a prescribed position, due to the object of the treatment, and the operability of the catheter in this case is very important. In view of operability, the catheter is generally made from a long and thin tubular member, and the thinness of the catheter itself, and especially the thinness of the front tip thereof, is extremely important, since the catheter must be operated from outside the body, via an insertion opening, and made to pass through curved regions inside the body or narrow and constricted internal parts of the body. In particular, the thinness and flexibility of the expandable body section and the vicinity thereof is important, since it is at the distal end of the catheter. This section is of course thin and soft, and since it is often inserted into curved regions, and the very softest part thereof rubs against the guide wire inserted through the inside thereof, it is demanded that the flexibility characteristics be continuous. A catheter provided with conventional cutters has reduced flexibility in the expandable body section, and improvement is required for application to curved affected regions, affected regions which have a curved region before them, calcified regions, stented regions affected by restenosis, or the like.

Moreover, in dilatation treatment, and in particular in a method for performing dilatation of an area that is to be dilated whilst making an incision therein, it is important that the expandable body size is determined with respect to the region for dilatation, whilst considering the diameter of the dilated region, the nature of the plaque, and its eccentricity, degree of calcification, position, and the like, by means of an ultrasonic endoscope, or the like, but in a dilatation catheter provided with conventional cutters, an expandable body with a very low expandability characteristics (compliance) must be selected, and hence it has been difficult to perform fine adjustment of the size of the expandable body by means of the degree of pressurization.

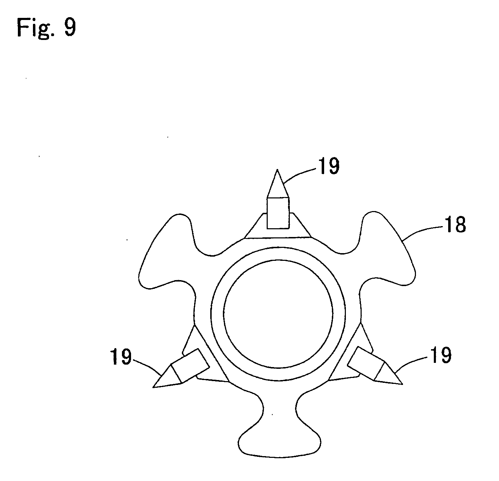

Furthermore, the dilatation catheter described in Japanese Patent No. 2591573 has also produced other problems, due to breaking off of the blades. When inserting the blades into the affected area for the purpose of dilatation, it is necessary to protect the blades so that they do not cause damage to healthy internal passages, other equipment used in the procedure, or the expandable body itself, and it is also necessary to protect the blades in a similar manner when removing it after dilatation. In the aforementioned dilatation catheter according to the prior art, in which blades may break off, it is necessary to adopt a special folded shape and method for the expandable body, whereby the expandable body assumes a bulky state, in order to protect the blades, as illustrated in Fig. 9, but since protection is achieved by causing the expandable body to assume a bulky, folded state, the protection from the blades will be lost if the folded up state is not maintained after dilatation, and hence healthy internal passages, and other equipment, or the expandable body itself, may become damaged. Moreover, in order that the expandable body effectively retains a bulky folded shape, the body must be formed with a certain degree of hardness, and this degrades the flexibility of the expandable body section, causing poor penetrability to the affected area, restricting expandability characteristics of the expandable body, or other such problems.

### SUMMARY OF THE INVENTION

In order to achieve the aforementioned objects, it is an object of a first aspect of the present invention to provide a dilatation catheter for use in surgery aimed at a dilatation operation of an internal body passage, whereby unintentional rupturing and damaging of internal body passages is prevented by inserting incisions in the region being dilated whilst dilating same, the dilatation catheter having excellent penetrability to the affected region and the expandable body having freely adjustable expandability characteristics.

As described above, in terms of operability and penetrability to the affected region, it is important that the front end portion of a dilatation catheter and the portion where the expandable body is attached are formed finely and flexibly, without having a large differential in hardness with respect to the other portions of the catheter. Moreover, in a method for dilating a region to be dilated whilst inserting incisions in same, in some cases, it is desirable to adopt a method whereby the size of the expandable body can be adjusted finely with respect to the dilated region. A conventional dilatation catheter provided with cutters has a structure wherein metallic blades forming atheroma cutters are attached by adhesive to an expandable body, and consequently the flexibility of the expandable body section is degraded, operability and penetrability to the affected region is poor, and it has been difficult to select the expandability characteristics of the expandable body. The object to be achieved by the present invention is to present a dilatation catheter of excellent operability, which has improved flexibility in the expandable body section, and allows the expandability characteristics of the expandable body to be selected freely.

In order to achieve the aforementioned object, the present invention is a dilatation catheter having a structure for performing dilatation whilst inserting incisions in the region being dilated, wherein the expandable body section is made finer and the flexibility thereof is improved, and moreover, the difference in hardness in the vicinity of the expandable body of the catheter is reduced to a minimum, thereby achieving a catheter of excellent operability, by means of modifying the structure, using selected positioning of materials and modifying the assembly method.

In other words, the dilatation catheter according to the present invention is a dilatation catheter consisting of a plurality of tubular members and a expandable body, desirably having a structure wherein both ends of the expandable body are fixed to the tubular members in the vicinity of the distal end of the catheter, and having a cutting blade structure installed on the tubular members in the vicinity of the expandable body.

Furthermore, in the aforementioned dilatation catheter, the cutting blade structure is installed on the tubular members at either end of the expandable body, in such a manner that the cutting blade of the cutting blade structure is positioned on the surface of the expandable body.

Moreover, in the aforementioned dilatation catheter, the cutting blade structure is fixed in such a manner that a tension is generated in the axial direction thereof.

Furthermore, in the aforementioned dilatation catheter, the cutting blade structure is constituted by a cutting blade and supporting sections for supporting the cutting blade and is installed in such a manner that the edge of the cutting blade is oriented in a perpendicular direction to the surface of the expanded expandable body, when the expandable body is caused to expand, and the cross-sectional shape of the supporting section in the direction perpendicular to the axis of the catheter is a flat shape which is elongated in the direction of the tangent to the circumference of the expandable body. Moreover, desirably, the material of the supporting section on the distal side of the cutting blade structure is set to a lower hardness than the material of the proximal side supporting section.

Moreover, in the aforementioned dilatation catheter, the expandability characteristics (compliance) of the expandable body is 0.80% per atmosphere (0.78%/MPa), or above.

By adopting the foregoing structure, the expandable body section of the catheter can be made sufficiently fine and flexible, and the expandability characteristics (compliance) of the expandable body can be controlled freely, thereby achieving the aforementioned objects.

Moreover, it is an object of a second aspect of the present invention to provide a dilatation catheter for use in surgery aimed at a dilatation operation of an internal body passage, whereby unintentional rupturing and damaging of internal body passages is prevented by inserting incisions in the region being dilated whilst dilating same, the dilatation catheter having excellent safety by improving the protection of the cutting blades using to perform the aforementioned incisions. It is a further object to provide a dilatation catheter which affords broader scope in the selection of the flexibility of the expandable body section, and has excellent penetrability to the affection region and allows free selection of the expandability characteristics of the expandable body.

As described previously, in a dilatation catheter which performs dilatation whilst inserting incisions in the region being dilated, it is possible from a safety viewpoint that the protection of the cutting blade is increased in such a manner that it does not cause harm to healthy parts of the internal body passages, or to other equipment involved, or to the expandable body of the catheter. Moreover, in terms of operability and penetrability to the affected region, it is important that the front end portion of a dilatation catheter and the portion where the expandable body is attached are formed finely and flexibly, without having a large differential in hardness with respect to the other portions of the catheter, and in a method for dilating a region to be dilated whilst inserting incisions in same, in some cases, it is desirable to adopt a method whereby the size of the expandable body can be adjusted finely with respect to the dilated region.

In an existing dilatation catheter provided with cutters, in order to protect the cutting blades, it is necessary to adopt a special folded over shape and method for the expandable body, in such a manner that the expandable body a bulky shape, but since the expandable body itself must have a certain degree of hardness in order to maintain this bulky folded over shape effectively, the flexibility of the expandable body section is impaired, the operability and penetrability to the affected region is poor and it has been difficult to select the expandability characteristics of the expandable body. The object to be achieved by the present invention is to provide a dilatation catheter having excellent safety by providing improved protection of the cutting blades, and it is a further object to provide a dilatation catheter which affords broader scope in the selection of the flexibility of the expandable body section, and has excellent penetrability to the affection region and allows free selection of the expandability characteristics of the expandable body.

The invention achieves the aforementioned objects by providing a dilatation catheter having a structure for performing dilatation whilst inserting incisions in the region being dilated by means of a cutting blade, which has excellent safety by improving the protection of the cutting blade.

In other words, the dilatation catheter according to the present invention is a dilatation catheter comprising a plurality of tubular members, an expandable body, and a cutting blade disposed in the vicinity of the expandable body, comprising protecting members for protecting the blade portion of the cutting blade, or alternatively, a dilatation catheter comprising a plurality of tubular members, an expandable body, and a cutting blade disposed in the vicinity of the expandable body, comprising protecting members having the functions of protecting the blade section of the cutting blade and exposing the blade section of the cutting blade when the cutting blade is pressed in the outward radial direction of the expandable body.

Moreover, the present invention is the aforementioned catheter, comprising a structure for substantially preventing contact between the blade edge and other objects by providing protecting members for protecting the blade section of the cutting blade in parallel with the blade section of the cutting blade on at least one side of the cutting blade, and furthermore, it is the aforementioned dilatation catheter having a structure for preventing contact between the blade edge and other objects by means of the protecting members for protecting the blade section of the cutting blade having a flat plate-shaped structure at least in the portion thereof disposed in parallel with the blade section of the cutting blade, and the flat plate-shaped portion having approximately the same height as the blade section of the cutting blade.

Furthermore, the present invention also provides the aforementioned dilatation catheter wherein the cutting blade is fixed to the expandable body, or the aforementioned dilatation catheter wherein a cutting blade structure comprising a cutting blade is installed on the tubular members in the vicinity of the expandable body, and hence excellent protection of the cutting blade is achieved, the scope of selection with respect to the flexibility of the expandable body section is broadened, and the aforementioned objects are achieved.

It is an object of a third aspect of the present invention to provide a dilatation catheter for use in surgery aimed at performing a dilatation operation of an internal body passage, whereby unintentional rupturing and damaging of internal body passages is prevented by inserting incisions in the region being dilated whilst dilating same, the dilatation catheter having excellent penetrability to the affected region and the expandable body having freely adjustable expandability characteristics. Moreover, it is a further object to provide a dilatation catheter having excellent safety by improving the protection of the cutting blade use to insert the incisions.

As described above, in terms of operability and penetrability to the affected region, it is important that the front end portion of a dilatation catheter and the portion where the expandable body is attached are formed finely and flexibly, without having a large differential in hardness with respect to the other portions of the catheter. Moreover, in a method for dilating a region to be dilated whilst inserting incisions in same, in some cases, it is desirable to adopt a method whereby the size of the expandable body can be adjusted finely with respect to the dilated region. A conventional dilatation catheter provided with cutters has a structure wherein metallic blades forming atheroma cutters are attached by adhesive to an expandable body, and consequently the flexibility of the expandable body section is degraded, operability and penetrability to the affected region is poor, and it has been difficult to select the expandability characteristics of the expandable body. The object to be achieved by the present invention is to present a dilatation catheter of excellent operability, which has improved flexibility in the expandable body section, and allows the expandability characteristics of the expandable body to be selected freely. Moreover, a dilatation catheter which inserts incisions in a region to be dilated by means of a cutting blade whilst dilating same requires means for protecting the cutting blade, and in the prior art, it is necessary to adopt a special folded over shape and method for the expandable body such that the expandable body assumes a bulky state in order to achieve this purpose, but since the expandable body itself must have a certain degree of hardness in order to maintain this bulky folded over shape effectively, the flexibility of the expandable body section is impaired, the operability and penetrability to the affected region is poor and it has been difficult to select the expandability characteristics of the expandable body, and if the folded over shape is not maintained, then the protection of the cutting blade is lost and the cutting blade will project out in the perpendicular direction to the axis of the catheter, thereby causing damage to the healthy internal body passages, the other equipment, or to the expandable body itself. The object to be achieved by the present invention is to provide a dilatation catheter having excellent safety by comprising improved protection of the cutting blade.

The present invention achieves the aforementioned objects by providing a new structure for a dilatation catheter, being a dilatation catheter having a structure for performing dilatation whilst inserting incisions in the region being dilated, wherein the incising capability of the dilatation catheter is improved by means of the selected material positions and assembly method, the dilatation catheter having excellent operability by improving the fineness and flexibility of the expandable body section, and minimizing the differential in hardness in the vicinity of the expandable body of the catheter.

In other words, the dilatation catheter according to the present invention is a dilatation catheter comprising a plurality of tubular members and an expandable body, having a structure wherein both ends of the expandable body are fixed to the tubular members in the vicinity of the distal end of the catheter, a cutting blade structure constituted by a cutting blade and supporting sections for supporting the cutting blades being installed on the tubular members in the vicinity of the expandable body by means of the supporting sections, and at least a portion of the supporting sections being made from a metallic material.

Here, desirably, the minimum thickness of the metallic material is in the range of 0.03 mm to 0.40 mm. Moreover, desirably, the cross-sectional shape of the supporting sections in a direction perpendicular to the axis of the catheter is a flat shape which is elongated in the direction of the tangent to the circumference of the expandable body. Furthermore, the metallic material is stainless steel or a superelastic alloy. Moreover, desirably, the metallic material is disposed by burying inside a resin.

Desirably, the metallic material is connected to the cutting blade, or alternatively, is disposed such that it passes through a hole portion provided in the cutting blade, and the supporting sections of the cutting blade structure are installed in the tubular members on either side of the expandable body and in the vicinity thereof, at least a portion of the proximal side supporting section only being made from metallic material.

The dilatation catheter according to the present invention is a dilatation catheter comprising a plurality of tubular members and an expandable body, having a structure wherein both ends of an expandable body are fixed to the tubular members in the vicinity of the distal end of the catheter, wherein a cutting blade structure constituted by a cutting blade and supporting sections for supporting the cutting blade is installed on the tubular members in the vicinity of the expandable body, by means of the supporting sections, the difference between the height of the supporting sections and the cutting blade in a perpendicular direction to the axis of the catheter being 0.15 mm or less, or alternatively, wherein a cutting blade structure constituted by a cutting blade and supporting sections for supporting the cutting blade is installed on the tubular members in the vicinity of the expandable body, by means of the supporting sections, the maximum value of the height of the cutting blade structure in the perpendicular direction to the axis of the catheter being 0.50 mm or less, and therefore it is possible to made the expandable body section of the catheter sufficiently fine and flexible, whilst providing excellent incising capability and safety, and it is also possible to control the expandability characteristics (compliance) of the expandable body freely, thereby achieving the aforementioned objects.

### BRIEF DESCRIPTION OF THE DRAWINGS

Fig. 1 is a simplified cross-sectional view showing the distal portion of a dilatation catheter comprising an expandable body of a dilatation catheter and a cutting blade structure installed on tubular members in the vicinity of the expandable body, relating to a first aspect of the present invention;
Fig. 2 is a simplified front view showing the distal portion of a dilatation catheter comprising an expandable body of a dilatation catheter and a cutting blade structure installed on tubular members in the vicinity of the expandable body, relating to a first aspect of the present invention, illustrated in Fig. 1, as viewed from the distal side in the axial direction;
Fig. 3 is a simplified cross-sectional diagram showing one example of a cutting blade structure relating to the first aspect of the invention;
Fig. 4 is a simplified front view showing one example of a cutting blade structure relating to the first aspect of the present invention, being a view of Fig. 3 from the distal side in the axial direction;
Fig. 5 is a simplified cross-sectional view showing a further example of the distal portion of a dilatation catheter comprising an expandable body of a dilatation catheter and a cutting blade structure installed on tubular members in the vicinity of the expandable body, relating to the first aspect of the present invention;
Fig. 6 is a simplified front view showing a further example of a dilatation catheter relating to the first aspect of the present invention:
Fig. 7 shows one example of a cutting blade in a cutting blade structure relating to the first aspect of the present invention, (a) being a simplified side view of the cutting blade and (b) being a simplified front view of the cutting blade;
Fig. 8 is a simplified cross-sectional view of one example of an expandable body in a folded over state in a dilatation catheter relating to the first aspect of the present invention;
Fig. 9 is a simplified cross-sectional view of an expandable body section in a folder over state in a dilatation catheter according to a comparative example;
Fig. 10 is an illustrative diagram giving a schematic illustration of a measurement system for demonstrating the benefits of the first aspect of the present invention;
Fig. 11 is a simplified cross-sectional view showing the distal portion of a dilatation catheter comprising an expandable body, a cutting blade disposed in the vicinity of the expandable body, and protecting members for protecting the blade section of the cutting blade, relating to a first embodiment of a second aspect of the present invention;
Fig. 12 is a simplified front view showing the distal portion of a dilatation catheter comprising an expandable body, a cutting blade disposed in the vicinity of the expandable body, and protecting members for protecting the blade section of the cutting blade, relating to the second aspect of the present invention, as illustrated in Fig. 11, viewed from the distal side in the axial direction;
Fig. 13 is a simplified cross-sectional view showing the distal portion of a dilatation catheter comprising an expandable body, a cutting blade structure comprising a cutting blade disposed in the vicinity of the expandable body, and protecting members for protecting the blade section of the cutting blade, relating to a second embodiment of the second aspect of the present invention;
Fig. 14 is a simplified front view showing the distal portion of a dilatation catheter comprising an expandable body, a cutting blade structure comprising a cutting blade disposed in the vicinity of the expandable body, and protecting members for protecting the blade section of the cutting blade, relating to the second aspect of the present invention, as illustrated in Fig. 13, viewed from the distal side in the axial direction;
Fig. 15 is a simplified cross-sectional view showing one example of a cutting blade structure relating to the second aspect of the present invention;
Fig. 16 is a simplified cross-sectional view showing a cutting blade of a cutting blade structure relating to the second aspect of the present invention;
Fig. 17 is a simplified cross-sectional view showing a cutting blade in a further example of a cutting blade structure relating to the second aspect of the present invention;
Fig. 18 is an illustrative view showing a state where an incision is made in an internal body passage by means of the dilatation catheter relating to the second aspect of the present invention;
Fig. 19 is a simplified front view showing a further example of a dilatation catheter relating to a first embodiment of a second aspect of the present invention;
Fig. 20 is a simplified front view showing a further example of a dilatation catheter relating to a second embodiment of a second aspect of the present invention;
Fig. 21 is a simplified cross'-sectional view showing one example of the expandable body portion of a dilatation catheter relating to a first embodiment of the second aspect of the present invention, in a folded over state;
Fig. 22 is a simplified cross-sectional view showing one example of the expandable body portion of a dilatation catheter relating to a second embodiment of the second aspect of the present invention, in a folded over state;
Fig. 23 is a simplified cross-sectional view showing the distal portion of a dilatation catheter comprising an expandable body, and a cutting blade structure installed on tubular members in the vicinity of the expandable body, relating to a third aspect of the present invention;
Fig. 24 is a simplified front view showing the distal portion of a dilatation catheter comprising an expandable body, and a cutting blade structure installed on tubular members in the vicinity of the expandable body, relating to a third aspect of the present invention, as illustrated in Fig. 23, as viewed from the distal side in the axial direction;
Fig. 25 is a simplified cross-sectional view showing one example of a cutting blade structure relating to the third aspect of the present invention;
Fig. 26 is a simplified cross-sectional view showing one example of a supporting section of a cutting blade structure relating to the third aspect of the present invention; and
Fig. 27 shows one example of a cutting blade structure relating to the third aspect of the present invention, (a) being a partial simplified plan view of a cutting blade structure and (b) being a partial simplified side view of a cutting blade structure.

### DESCRIPTION OF THE PREFERRED EMBODIMENTS

Below, the present invention is described in detail with reference to the accompanying drawings.

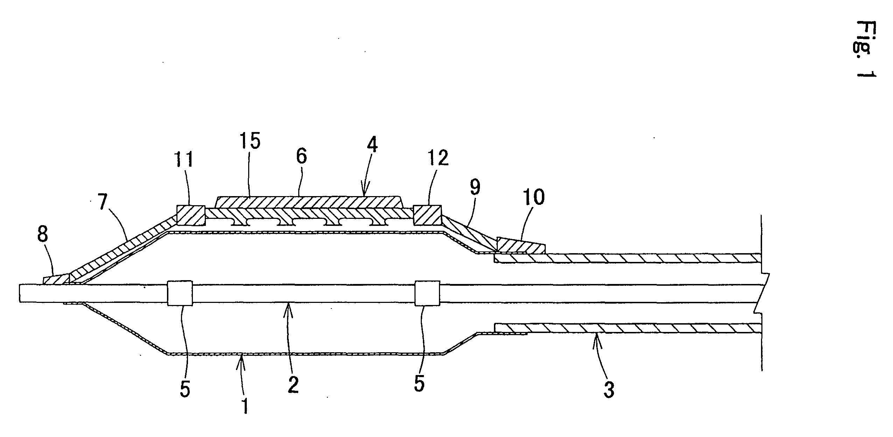

Firstly, an embodiment of a dilatation catheter relating to a first aspect of the invention is described on the basis of Fig. 1 to Fig. 10. The present invention is a dilatation catheter constituted by a plurality of tubular members, and desirably, in a dilatation catheter having a structure wherein the respective ends of an expandable body are fixed to the tubular members in the proximity of the distal end of the catheter, the dilatation catheter comprises a cutting blade structure installed on the tubular members in the vicinity of the expandable body. Here, for the tubular members on which the cutting blade structure 4 is installed in the dilatation catheter according to the present invention, in view of the objects of the present invention, it is possible to adopt various tubular members apart from the expandable body 1 forming the dilatation catheter, for example, a tubular member 2 for passing a guide wire, a tubular member 3 forming the outer face of the catheter, a tip section formed by fixing the expandable body 1 and the tubular member 2 for passing the guide wire in a concentric fashion at the front tip of the catheter, and the like. Moreover, the cutting blade structure 4 and the various tubular members do not necessarily have to be installed together in a direct fashion, but rather, may be assembled indirectly, by means of various types of configurations.

Fig. 1 is a simplified sectional drawing of one example of the vicinity of an expandable body, being the distal portion of a dilatation catheter relating to the present invention, and it comprises an expandable body 1, a tubular member 2 for passing a guide wire, a tubular member 3 forming an outer face of the catheter, and a cutting blade structure 4. In Fig. 1, the tubular member 2 for passing the guide wire is disposed passing through the interior of the expandable body 1, and a tip section is formed by fixing same in a concentric fashion with the expandable body 1 at the foremost tip of the catheter. The expandable body 1 is connected to the tubular member 3 forming the outer face of the catheter, at the other end thereof. An X-ray shielding ring 5 may be fixed to the tubular member 2 for passing the guide wire. The cutting blade structure 4 is constituted by a cutting blade 6, a distal side supporting section 7, and a proximal side supporting section 9, the distal side supporting section 7 being fixed to the tip section consisting of the tubular member 2 for passing the guide wire, at a connecting section 8, and being fixed to the cutting blade 6 at a connecting section 11, and the proximal side supporting section 9 being fixed at a connecting section 10 to the tubular member 3 constituting the outer face of the catheter and also being fixed to the cutting blade 6 at a connecting section 12.

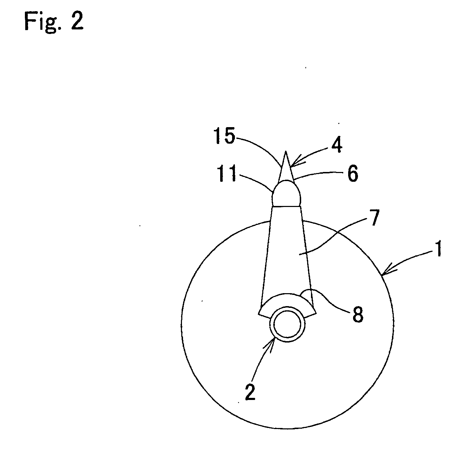

Fig. 2 is a simplified front view of Fig. 1, as view from the distal side in the axial direction of the dilatation catheter. In Fig. 2, the tubular member 2 for passing the guide wire forms a tip section by being fixed in a concentric fashion with the expandable body 1 at the foremost end of the catheter, and the supporting section 7 at the distal side of the cutting blade structure 4 is fixed at the connecting section 8 to the tip section formed by the tubular member 2 for passing the guide wire. Although the cutting blade structure 4 may be fixed to one of the tubular members fixed to either end of the expandable body 1, in order that the cutting blade structure 4 is held in a stable fashion, desirably, it should be installed on the tubular members 2, 3 at either end, as illustrated in Fig. 1.

Moreover, from the objective of cutting open the affected region during dilatation, it is desirable that the cutting blade 6 of the cutting blade structure 4 be positioned in the expandable body section, as illustrated in Fig. 1, and moreover, it is desirable that it be disposed in the central tubular portion of the expandable body 1, in order that the cutting blade structure 4 be held readily in a stable manner. In this case, desirably, X-ray shielding rings 5, 5, are fixed to the tubular member 2 in such a manner that they indicate the distal end and the near end of the cutting blade 6.

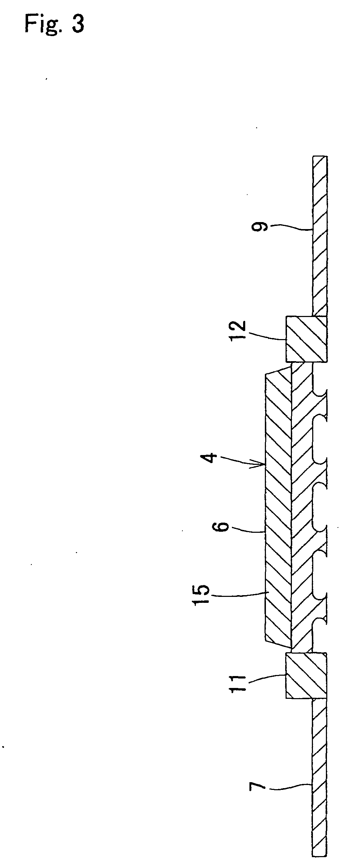

Moreover, the structure of the cutting blade structure 4 may be constituted by a single component only, such as a cutting blade 6, for example, but in order to facilitate the fixture thereof to the dilatation catheter, it is desirable that the cutting blade structure 4 be constituted by a cutting blade 6 and supporting sections 7, 9 for supporting the cutting blade 6, as illustrated in Fig. 1 and Fig. 3, for example.

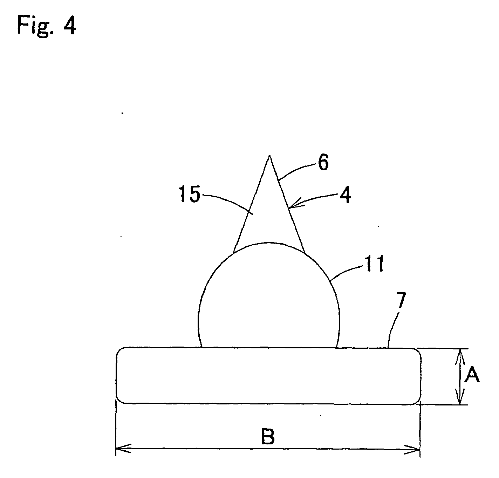

The cutting blade structure 4 is desirably installed in such a manner that that the cutting face of the cutting blade 6 faces perpendicularly with respect to the surface of the expandable body 1, and for this purpose, it is desirable that the cross-sectional shape of the supporting sections 7, 9 in the perpendicular direction to the catheter axis be a flat shape which extends in the direction of the tangent to the circumference of the lumens, in other words, that the length of the supporting section 7 in the B direction be comparatively larger than the length thereof in the direction A, as illustrated in Fig. 4. By adopting a structure of this kind, the surface area of the supporting sections 7, 9 contacting with a single surface of the expandable body 1 is increased, and consequently, the angle of the cutting blade 6 can be maintained in a stable fashion during dilatation.

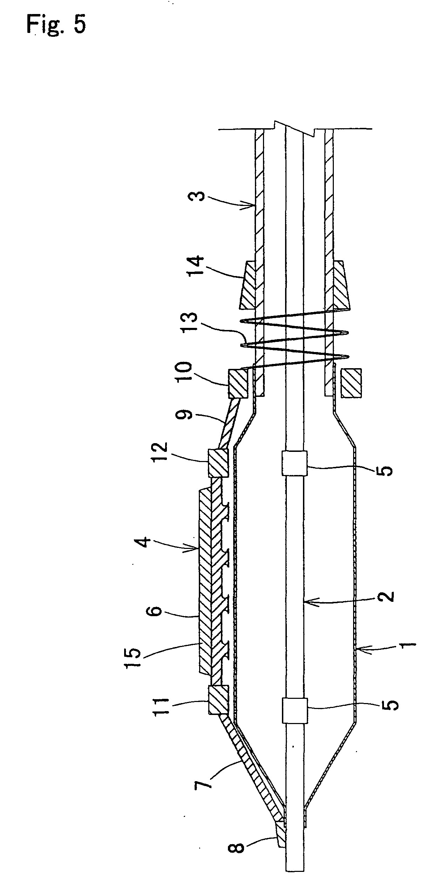

Desirably, when installing the cutting blade structure 4 on the catheter, it should be fixed in such a manner that a tensile force acting in the axial direction of the catheter is generated in the cutting blade structure 4. By adopting a structure of this kind, the cutting blade structure 4 can be aligned with the catheter axis and the profile thereof can be reduced, and moreover, it is possible to cause the cutting blade structure 4 to be disposed in alignment with the expandable body 1 during dilatation of the expandable body 1. In order to generate a tensile force, it is possible to manufacture the cutting blade structure 4 from a material having some degree of elasticity, such that it can deform elastically in the axial direction, and affix the cutting blade structure 4 in a state where it is deformed elastically by being pulled in the axial direction. In a further method, the cutting blade structure 4 may be connected indirectly to the tubular member 3 forming the outer face of the catheter, at a tubular member connecting section 14, by means of a coil shaped elastic member 13, as illustrated in Fig. 5, for example, an axial tension being generated in the cutting blade structure 4 by means of the stress occurring in the coil shape elastic member 13.

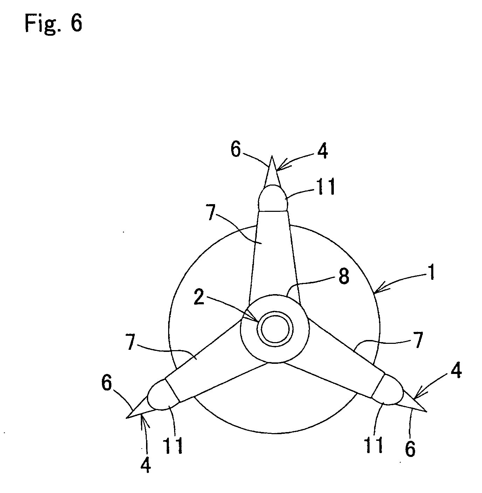

There are no particular restrictions on the number of cutting blade structures 4 which can be installed, and a plurality of cutting blade structures 4 may be installed. Fig. 6, for example, shows a simplified front view of a dilatation catheter fitted with three cutting blade structures 4, as viewed from the distal side in the axial direction. The tubular member 2 for passing the guide wire forms a tip section by being fixed in a concentric fashion with the expandable body 1 at the foremost end of the catheter, and the three cutting blade structures 4 are affixed to the tip section formed by the tubular member 2 for passing the guide wires at the connecting section 8, in such a manner that they are disposed at equidistant intervals about the circumference of the expandable body 1. As illustrated in Fig. 6, when a plurality of cutting blade structures 4 are provided, desirably, the respective cutting blade structures 4 are installed in such a manner that they are disposed at equidistant intervals about the circumference of the expandable body 1 when the expandable body 1 is expanded.

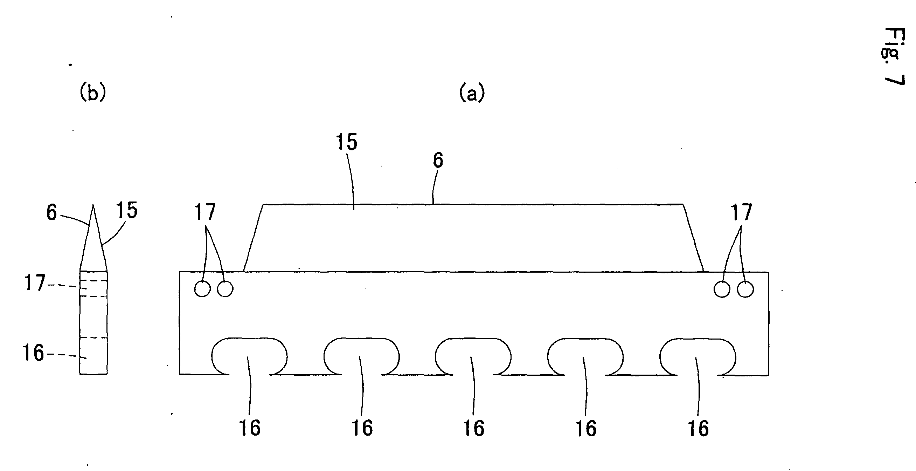

Although it is possible to achieve the objects by using resin as the material for the cutting blade 6 of the cutting blade structure 4, in some cases the use of metal is more desirable, in view of the fact that it allows the blade section 15 to be adjusted to high sharpness and a desired hardness, and stainless steel is desirable as the metal. Moreover, although there is no particular restriction on the shape of the blade, as shown in Fig. 7, it may be constructed with a plurality of approximately elliptical shaped cutaway portions 16 in order to increase the flexibility on the opposite side to the blade section 15. Furthermore, desirably, holes 17 are formed on the end portions in order to secure the connection with the supporting sections 7, 9 supporting the cutting blades 6.

If the cutting blade structure 4 is constituted by a cutting blade 6 and supporting sections 7, 9, then it is desirable that the material of the supporting sections 7, 9 be resin, since this allows the cutting blade structure as a whole to be constructed flexibly, and also allows superior manufacturability and assembly characteristics. In particular, it is desirable that the material of the supporting sections 7, 9 be a resin which can be fused with the tubular members 2, 3 of the catheter, from the viewpoint of improving manufacturing and assembly characteristics and allowing the fixing sections to be formed with a low profile. In this case, there may be situations where it is preferable if at least the portion of the tubular members 2, 3 of the catheter where they are fixed to the cutting blade structure 4 is formed with a multiple-layer structure of two or more layers, wherein a material which can be fused with the cutting blade structure 4 is disposed on the outermost layer, in view of the fact that this allow fusion forming whilst maintaining other characteristics required in the catheter. For example, if the whole of the tubular member 2 for passing the guide wire is made from polyethylene in order to maintain good slidability of the guide wire, and a resin which can be fused with the supporting section 7 of the cutting blade structure 4 is disposed at least on the outermost layer of the portion thereof which forms the front end tip, then it is possible to fuse the elements together whilst maintaining slidability characteristics.

Moreover, by selecting the material for the distal side supporting section 7 of the cutting blade structure 4 in such a manner that it has a lower hardness than the material of the proximal side supporting section 9, it is possible to achieve a dilatation catheter which increases in flexibility gradually, towards the front tip thereof. The expandability characteristics (compliance) of the expandable body 1 are expressed as a rate of increase in the diameter with respect to the applied pressure, and should be measured by calculating the rate of increase of the diameter with respect to the applied pressure in the range of the operating pressure, but it is also possible to achieve a calculation with low error by taking the rate of increase between the diameter at a nominal pressure, and the diameter at a maximum recommended pressure, and dividing this figure by the pressure differential between the nominal pressure and the maximum recommended pressure, in order to determine a rate of increase per atmosphere. Desirably, the expandability characteristics (compliance) of the expandable body 1 are 0.80% per atmosphere (0.78%/MPa), since this readily permits suitable change in the diameter of the expandable body 1 at the applied pressures used during surgery.

### (Embodiments of the first aspect of the invention)

Below, more specific embodiments and comparative examples relating to the present invention are described in detail, but the following embodiments do not limit the scope of the present invention in any way.

### (First embodiment)

Three cutting blade structures 4, each formed by connecting a distal side supporting section 7 (made from polyimide elastomer of Shore hardness 40D, and having a cross-sectional shape with a length of 0.20 mm in the A direction and a length of 0.50 mm in the B direction, as shown in Fig. 4), and a proximal side supporting section 9 (made from polyimide elastomer of Shore hardness 72D, and having a cross-sectional shape with a length of 0.20 mm in the A direction and a length of 0.50 mm in the B direction, as shown in Fig. 4), to a stainless steel cutting blade 6, were installed on the tubular members 2, 3 in the vicinity of the expandable body of a dilatation catheter using a polyimide elastomer expandable body 1 having a nominal expansion diameter of 3.0 mm, and expandability characteristics (compliance) of 0.80% (0.78%/MPa), and a fast-switching type dilatation catheter for use in coronary arteries was fabricated having the catheter distal portion illustrated in Fig. 5.

The installation of each cutting blade structure 4 onto the tubular members 2, 3, is performed by forming the outermost layer of the tubular member 2 for passing a guide wire with polyimide elastomer, at the distal side thereof, and then securing the distal side supporting section 7 by fusing at the tip section and the outer face of the outermost layer of the tubular member 2 for passing the guide wire. Moreover, by connecting the proximal side supporting section 9 to a coil shaped elastic body 13, at the proximal side, and fixing the coil shaped elastic member 13 with the tubular member 3 forming the outer face of the catheter, at a tubular member connecting section 14, consequently, the cutting blade structures 4 and the tubular member 3 forming the outer face of the catheter are fixed in such a manner that a tension in the axial direction is generated in the cutting blade structures 4.

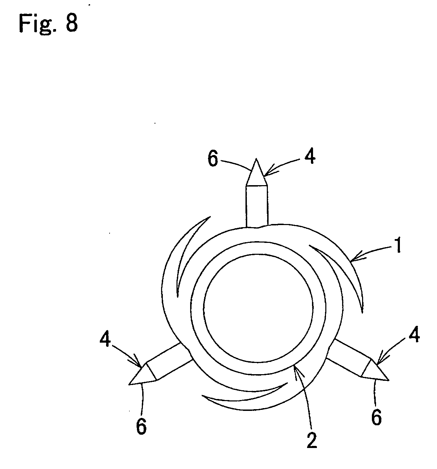

At the start of surgery, the expandable body 1 is folded over the perimeter of the tubular member 2 passing through the interior thereof, and the catheter is presented in a state where the cutting blade structures 4 are disposed in the vicinity of the outer circumference of the folded expandable body 1. Fig. 8 shows a simplified cross-sectional view, in a direction perpendicular to the axis of the catheter, of the central portion of the expandable body according to the present embodiment, when the expandable body 1 is folded up. The maximum diameter of the expandable body portion according to the present embodiment was 1.35 mm, and the bending rigidity according to a three-point bending test was 57 N·mm².

### (Second embodiment)

Three cutting blade structures 4, each formed by connecting a distal side supporting section 7 (made from polyimide elastomer of Shore hardness 40D, and having a cross-sectional shape with a length of 0.20 mm in the A direction and a length of 0.50 mm in the B direction, as shown in Fig. 4), and a proximal side supporting section 9 (made from polyimide elastomer of Shore hardness 72D, and having a cross-sectional shape with a length of 0.20 mm in the A direction and a length of 0.50 mm in the B direction, as shown in Fig. 4), to a stainless steel cutting blade 6, were installed on the tubular members 2, 3 in the vicinity of the expandable body of a dilatation catheter using a polyimide elastomer expandable body 1 having a nominal expansion diameter of 3.0 mm, and expandability characteristics (compliance) of 1.25% per atmosphere (1.23%/MPa), and a fast-switching type dilatation catheter for use in coronary arteries was fabricated having the catheter distal portion illustrated in Fig. 5.

The installation of each cutting blade structure 4 onto the tubular members 2, 3, was performed by forming the outermost layer of the tubular member 2 for passing a guide wire with polyimide elastomer, at the distal side thereof, and then securing the distal side supporting section 7 by fusing at the tip section and the outer face of the outermost layer of the tubular member 2 for passing the guide wire. Moreover, by connecting the proximal side supporting section 9 to a coil shaped elastic body 13, at the proximal side, and fixing the coil shaped elastic member 13 with the tubular member 3 forming the outer face of the catheter, at a tubular member connecting section 14, consequently, the cutting blade structures 4 and the tubular member 3 forming the outer face of the catheter are fixed in such a manner that a tension in the axial direction is generated in the cutting blade structures 4.

At the start of surgery, the expandable body 1 is folded over about the perimeter of the tubular member 2 passing through the interior thereof, and the catheter is presented in a state where the cutting blade structures 4 are disposed in the vicinity of the outer circumference of the folded expandable body 1. Fig. 8 shows a simplified cross-sectional view, in a direction perpendicular to the axis of the catheter, of the central portion of the expandable body according to the present embodiment, when the expandable body 1 is folded up. The maximum diameter of the expandable body portion according to the present embodiment was 1.35 mm, and the bending rigidity according to a three-point bending test was 55 N·mm².

### (Comparative example 1)

Comparative example 1 is a commercial dilatation catheter constituted by three atheroma cutters 19 (metal blades attached to an expandable body by polyurethane adhesive) installed on the outer face of an expandable body 18. Fig. 9 shows a simplified cross-sectional view, in a direction perpendicular to the axis of the catheter, of the central portion of the expandable body according to this comparative example when the expandable body 18 is folded up. The expandability characteristics (compliance) of the expandable body section of comparative example 1 were 0.67% per atmosphere (0.66%/MPa), the maximum diameter was 1.52 mm, and the bending rigidity according to a three-point bending test was 383 N·mm².

### (Evaluation)

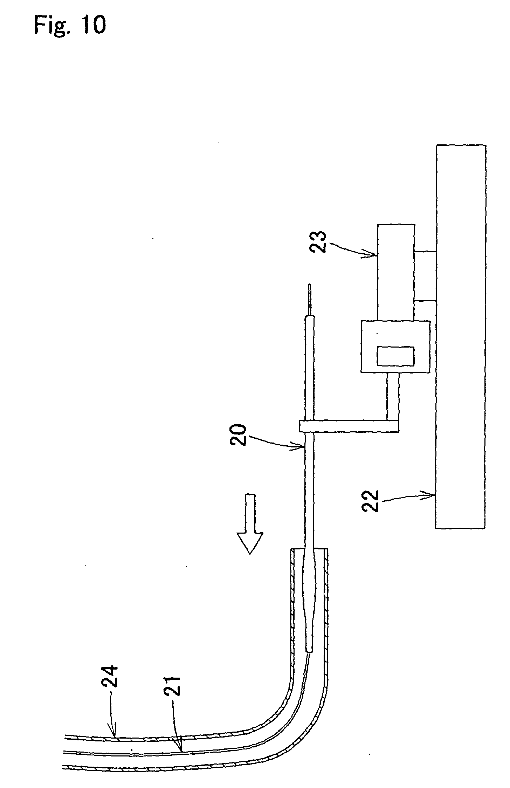

In an evaluation system as illustrated schematically in Fig. 10, in other words, an imitation of a curved internal body passage 24 fabricated from a polyethylene tube having an internal diameter of 2.0 mm and bent through 90º at a bending rate of 7 mm, inside which a guide wire 21 was disposed and through which a physiological saline solution adjusted to a temperature of 37QC was circulated, a dilatation catheter 20 incorporating the first and second embodiments, and comparative example 1, was advanced at a uniform speed along the guide wire 21, and the load applied to the dilatation catheter 20 'when the expandable body portion passed through the curved section was measured. For this purpose, the shaft of the dilatation catheter 20 was attached to a force gauge 23 provided in a linearly movable fashion on a slide table 22, and the value indicated by the force gauge 23 when advancing at the uniform rate was read off. The results are illustrated in Table 1, and in the case of the first and second embodiments of the present invention, the load when passing the expandable body section through the curved imitation internal body passage 24 was lower than that of the comparative example, thus demonstrating that the expandable body section is flexible and that the dilatation catheter provides excellent operability.

**Table 1**

| Measurement results based on measurement system for illustrating benefits of the first aspect of the invention | |
|---|---|
| | Peak load (N) |
| First embodiment | 0.21 |
| Second embodiment | 0.17 |
| Comparative example 1 | 0.60 |

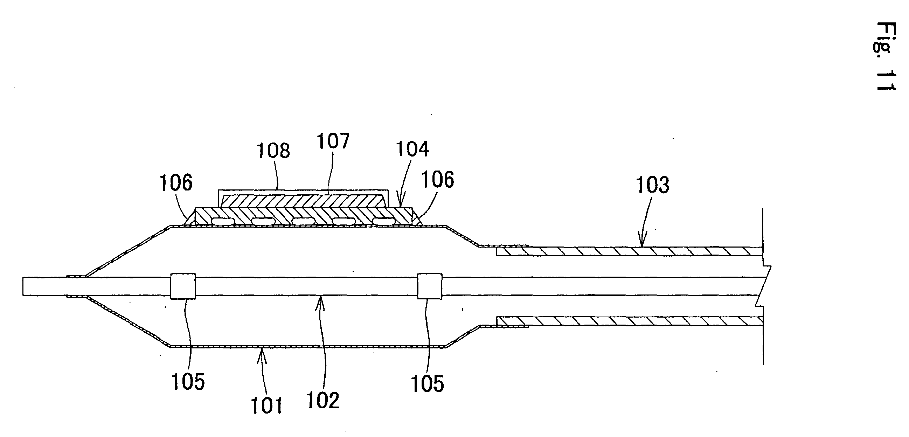

Next, an embodiment of a dilatation catheter relating to a second aspect of the invention is described on the basis of Fig. 11 to Fig. 22. The present invention is a dilatation catheter comprising a plurality of tubular members, an expandable body, and a cutting blade structure disposed in the vicinity of the expandable body, characterized in that it comprises a protecting member for protecting the blade portion of the cutting blade.

Fig. 11 is a simplified cross-sectional diagram showing a first embodiment of a dilatation catheter relating to the present invention, and it illustrates the state of implementation of an expandable body 101, a tubular member 102 having a lumen for passing a guide wire, a tubular member 103, having a lumen for introducing pressure to the expandable body, which forms the outer face of the catheter, a cutting blade 104 fixed and installed in a contacting fashion to the expandable body by means of adhesive 6 in the vicinity of the expandable body, and a protecting member 108 for protecting the blade section 107 of the cutting blade. In Fig. 11, the tubular member 102 having a lumen for passing the guide wire is disposed passing through the inside of the expandable body 101, and it is formed into a tip section by being fixed in a concentric fashion with the expandable body at the foremost end of the catheter. The expandable body 101 is connected to the tubular member 103 forming the outer face of the catheter, at the other end thereof. X-ray shielding rings 105 may be fixed onto the tubular member 102. Furthermore, the cutting blade 104 and the plate-shaped protecting members 108 which protect the blade section 107 of the cutting blade are fixed onto the expandable body 101 by means of an adhesive 106.

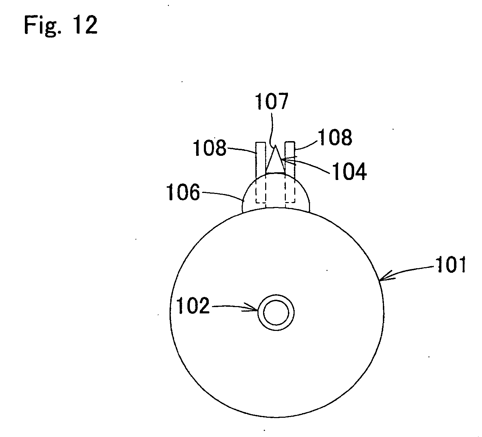

Fig. 12 is a simplified front view of Fig. 11, as viewed from the distal side in the axial direction of the dilatation catheter. In Fig. 12, the tubular member 102 having a lumen for passing the guide wire is formed into a tip section by being fixed in a concentric fashion with the expandable body at the foremost end of the catheter. The plate-shaped protecting members 108 for protecting the blade section 107 of the cutting blade 104 are fixed to the expandable body by the adhesive 106 in a state where they are disposed in parallel with and in tight contact with the cutting blade 104. Here, disposing the members in parallel with the cutting blade 104 means that the interval between the cutting blade 104 and the protecting members 108 is virtually uniform in the axial direction of the dilatation catheter. However, desirably, they form tight contact with the blade and no interval is formed therebetween.

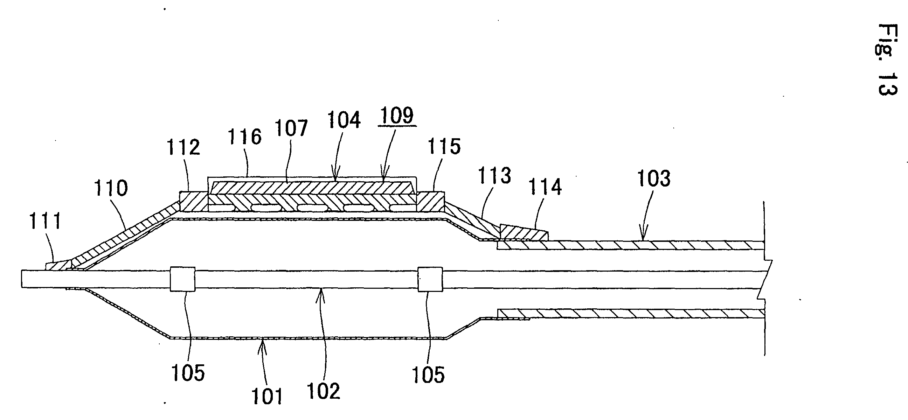

Fig. 13 is a simplified cross-sectional view showing a second embodiment of a dilatation catheter relating to the present invention, and it illustrates one example of the vicinity of a expandable body being the distal portion of a catheter constituted by an expandable body 101 of a dilatation catheter relating to the present invention, a tubular member 102 having lumens for passing a guide wire, a tubular member 103 having a lumen for introducing pressure to the expandable body 101, which forms the outer face of the catheter, and a cutting blade structure 109. Here, in the present embodiment, the cutting blade 104 and protecting members 116 are not installed directly on the expandable body 101, but rather, a structure is adopted whereby they are installed on the tubular members 102, 103 in the vicinity of the expandable body 101, and all of the members including the cutting blade 104 and the protecting members 116, and the composition for installing these on the tubular members in the vicinity of the expandable body, is termed the "cutting blade structure 109".

In Fig. 13, the tubular member 102 having a lumen for passing the guide wire is disposed passing along the inside of the expandable body 101, and is formed into a tip section by being fixed in a concentric fashion with the expandable body 101 at the foremost end of the catheter. The expandable body 101 is connected to the tubular member 103 forming the outer face of the catheter, at the other end thereof. X-ray shielding rings 105 may be fixed to the tubular member 102. The cutting blade structure 109 is constituted by a cutting blade 104, distal side supporting section 110, and proximal side supporting section 113, the distal side supporting section 110 being fixed to the tip section formed by the tubular member 102 at a connecting section 111 and being fixed to the cutting blade 104 at a connecting section 112, and the proximal side supporting section 113 being fixed to a tubular member 103 at the connecting section 114 and being fixed to the cutting blade 104 at the connecting section 115. Plate-shaped protecting members 116 for protecting the blade section 107 of the cutting blade 104 are fixed to the cutting blade 104 in a state whereby they enclose the cutting blade 104 from either side thereof.

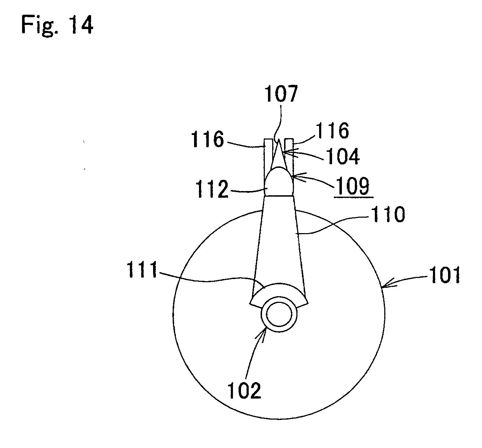

Fig. 14 is a simplified front view of Fig. 13, as viewed from the distal side in the axial direction of the dilatation catheter. In Fig. 14, the tubular member 102 having a lumen for passing the guide wire is formed into a tip section by being fixed in a concentric fashion with the expandable body at the foremost end of the catheter, and the supporting section 110 on the distal side of the cutting blade structure 109 is fixed to the tip section formed by the tubular member 102, at the connecting section 111. There are no particular restrictions on the shape or positioning of the protecting members 116, provided that they enable suitable protection of the blade section 107, but desirably, the shape and position should have a function whereby the blade section 107 is exposed when the cutting blade 104 is pressed in the outward radial direction of the expandable body 101.

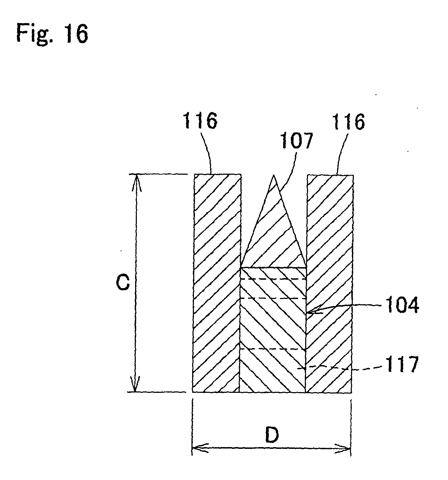

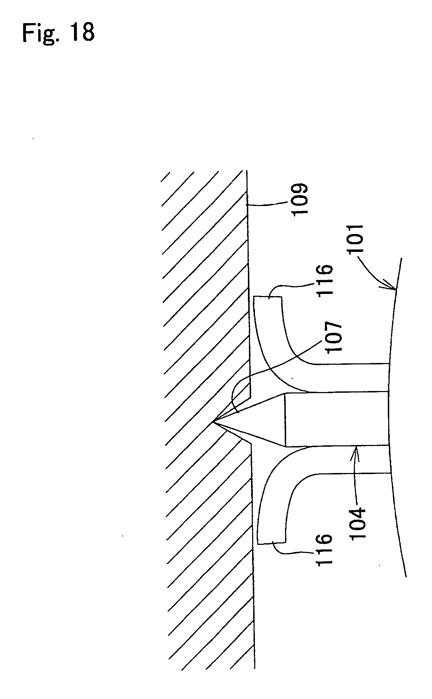

Below, the cutting blade 104 and protecting members 116 are described in detail with reference to the sectional view in Fig. 16. Plate-shaped protecting members 116 for protecting the blade section 107 are fixed to either side of the cutting blade 104 in a parallel fashion whereby they enclose the cutting blade 104 from either side thereof. In a state prior to expansion, due to the presence of the protecting members 116, the blade section 107 is protected in such a manner that it does not touch any other objects, and hence it does not cause damage to any objects other than the desired objects. When the expandable body 101 is caused to expand in the desired location, the cutting blade 104 which has been protected by these protecting members 116 is pressed strongly against the wall of the internal body passage in the outward radial direction of the expandable body 101, but as illustrated in Fig. 18, the protecting members 116 are bent over and the blade section 107 is exposed and is hence able to make an incision in the internal body passage 119. As described above, the protecting members 116 for protecting the blade section 107 of the cutting blade 104 are positioned at least in parallel with the blade section 107 on one side of the cutting blade 104 and have a structure for preventing the blade edge from substantially making contact with other objects, which is desirable in terms of achieving the aforementioned functions by means of a relatively simple composition, and preferably, the hardness of the protecting members 116 achieves a balance between preventing the blade section 107 from approaching other objects, and bending over and exposing the blade section 107 when pressure is applied thereto. Consequently, it is desirable to use a resin containing rubber material as the material for the protecting members 116, and more desirably, a thermoplastic elastomer material, such as polyamide elastomer. The material for the protecting members 108 described above is similar to that of the protecting members 116. Furthermore, the height of the protecting members 108, 116 should take account of the material and shape forming the protecting members 108, 116, and desirably, it is adjusted to a height whereby the blade section 104 is exposed when the blade section 104 is pressed in the outward radial direction of the expandable body 101 during expansion of the expandable body, but whereby it is not exposed simply by contact with the walls of the internal body passage, when not expanded. On the other hand, although a simple, long and plate-shaped structure is conceived for the protecting members 108, 116, these members do not necessarily have to be of a simple structure, but may also, for example, be of a plate shape formed with zigzag-shaped cuts, or the like, in a portion of the protecting members 108, 116. In this case, the distal portion of the dilatation catheter consisting of a cutting blade 104, protecting members 108, 116, and an expandable body 101, can be formed in a more flexible manner.

Furthermore, although there is no particular restriction on the positioning of the cutting blade 104, it may be fixed to the expandable body 101, as illustrated in the first embodiment described above, or the cutting blade 104 may be composed as a portion of the cutting blade structure 109, with the cutting blade structure 109 being installed on the tubular members 102, 103 in the vicinity of the expandable body, as illustrated in the second embodiment. The latter case is more desirable in terms of allowing the catheter to be formed in a more flexible manner.

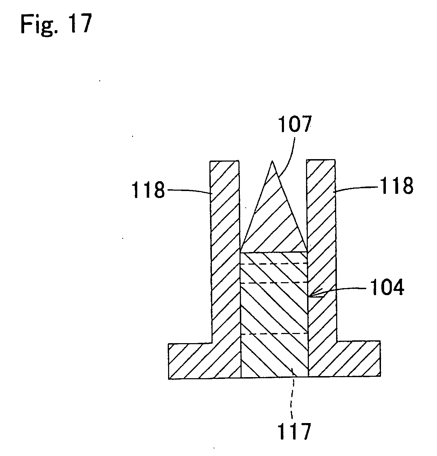

In the case of the second embodiment, it is permissible for the cutting blade structure 109 to be fixed to one of the tubular members of the expandable body 101, but in order for the cutting blade structure 109 to be held in a stable manner, it is desirable that it be attached to the tubular members 102, 103 at either end of the expandable body 101, as illustrated in Fig. 13. Moreover, it is desirable that the cutting blade structure 109 and cutting blade 104 be disposed in the region of the expandable body, as illustrated in Fig. 13, in view of the object of cutting open the affected region during dilatation, and furthermore, it is more desirable that they be disposed in the central tubular region of the expandable body 101, so that the cutting blade structure 109 can be readily held in a stable manner. In this case, desirably, X-ray shielding rings 105 are fixed to the tubular member 102, in order to indicate the distal end and near end positions of the cutting blade 104. Desirably, the cutting blade structure 109 is installed in such a manner that the blade edge of the cutting blade 104 assumes an approximately perpendicular attitude with respect to the outward radial direction from the line of axis of the dilated expandable body 101, when the expandable body 101 is caused to expand, and desirably, it should be located stably in this state. For this purpose, as illustrated in Fig. 16, it is desirable that the width D of the base portion of the cutting blade 104 in the cutting blade structure 109 be at least one half or more of the height C of the cutting blade 104, from the viewpoint of maintaining the angle of the blade section 107 stably during dilatation, and it is yet more desirable that the protecting members 118 have a structure whereby they are larger in the width direction at the base portions thereof, as illustrated in Fig. 17.

Furthermore, in the second embodiment, when installing the cutting blade structure 109 onto the catheter, it is desirable that it be fixed in such a manner that a tension is generated in the cutting blade structure 109 in the axial direction of the catheter, since this helps to align the cutting blade structure 109 with the axis of the catheter, thus lowering the profile thereof, as well as helping to position the cutting blade structure 109 in alignment with the expandable body 101. As a method for generating tension, it is possible to fabricate the cutting blade structure 109 from a material having sufficient elasticity to permit elastic deformation thereof in the axial direction, and to fix it in an elastically deformed state by pulling it in the axial direction. In a further method, for example, as described in relation to the embodiments of the first aspect of the invention, it is possible to connect the cutting blade structure 109 to the catheter tubular member 103 by means of a coil shaped elastic member 13, and a coil and tubular member connecting section 14, an axial tension being generated in the cutting blade structure 109 by means of the stress occurring in the coil-shaped elastic member 13.

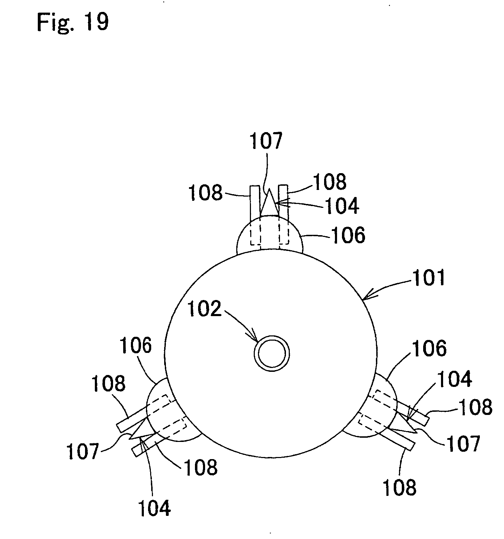

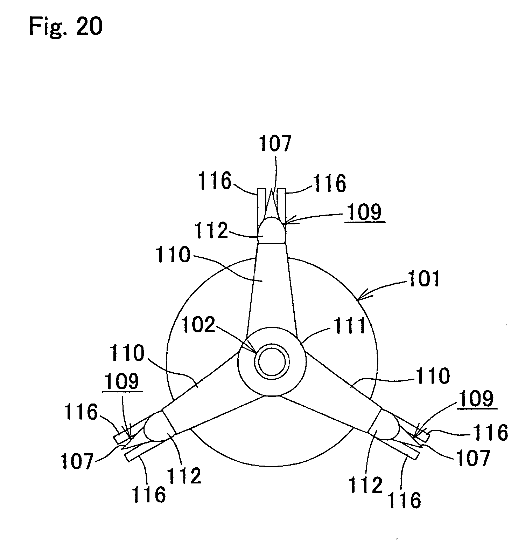

The number of cutting blades 104 installed on the dilatation catheter may be a plurality: Fig. 19 is a simplified front view of a dilatation catheter wherein three cutting blades 104 are fixed to an expandable body 101, as viewed from the distal side in the axial direction, and Fig. 20 is a simplified front view of a dilatation catheter as viewed from the distal side in the axial direction, in which cutting blades 104 are constituted as part of a cutting blade structure 109, and three cutting blade structures 109 are installed on the tubular members 102, 103 in the vicinity of an expandable body. In Fig. 19, the tubular member 102 having a lumen for passing the guide wire is formed into a tip section by being fixed in a concentric fashion with the expandable body 101 at the foremost end of the catheter, and the three cutting blades 104 are fixed to the expandable body 101 by adhesive 106, in such a manner that the three cutting blades 104 are disposed at equidistant intervals about the circumference of the expandable body 101. In Fig. 20, the tubular member 102 having a lumen for passing the guide wire is formed into a tip section by being fixed in a concentric fashion with the expandable body 101 at the foremost end of the catheter, and the three cutting blade structures 109 are fixed to the tip section formed by the tubular member 102 at a connecting section 111, in such a manner that the three cutting blade structures 109 are disposed at equidistant intervals about the circumference of the expandable body 101. When a plurality of cutting blades 104 or a plurality of cutting blade structures 109 including cutting blades 104 are provided, as illustrated in Fig. 19 and Fig. 20, it is desirable that the respective cutting blades 104 or the respective cutting blade structures 109 comprising cutting blades 104 are installed in such a manner that they are disposed at equidistant intervals about the circumference of the expandable body 101, when the expandable body 101 is expanded.

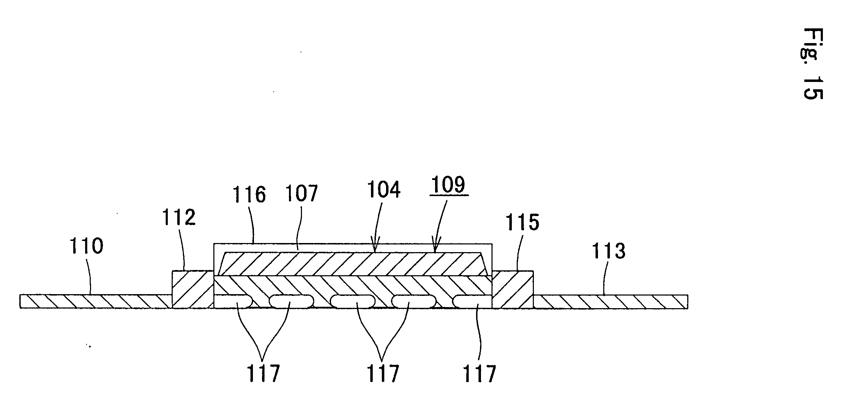

Whilst the use of resin as the material for the cutting blade 104 is satisfactory for achieving the objectives, in some cases the use of metal is more desirable, in view of the fact that it allows the blade section 107 to be adjusted to high sharpness and a desired hardness, and stainless steel is desirable as the metal. Moreover, although there is no particular restriction on the shape of the blade, as shown in Fig. 15, it may be constructed with a plurality of approximately elliptical shaped cutaway portions 117 on the opposite side to the blade section 107, in order to increase flexibility for facilitating the fixing of protecting member 116. Furthermore, desirably, holes are formed on the end portions as illustrated in Fig. 7 described above, in order to connect the cutting blade 104 to the cutting blade structure 109 and in order to secure the connection with the supporting sections 110, 113 supporting the blade section 107. If the cutting blade structure 109 is constituted by a cutting blade 104, and supporting sections 110, 113 which support the cutting blade 104, then the material of the supporting section 110, 113 should desirably be a resin, since this allows excellent manufacturability and assembly characteristics, whilst permitting maximum possible flexibility in the overall composition of the cutting blade structure 109. Moreover, in view of being able to achieve excellent manufacturability and assembly characteristics, and hence forming the fixing section to have a low profile, it is especially desirable that the material of the supporting sections 110, 113 be a resin which can be fused to the tubular members 102, 103 of the catheter.

### (Embodiments of the second aspect of the invention)

Below, more specific embodiment and comparative examples relating to the present invention are described in detail, but the following embodiments do not limit the scope of the present invention in any way.

### (Third embodiment)

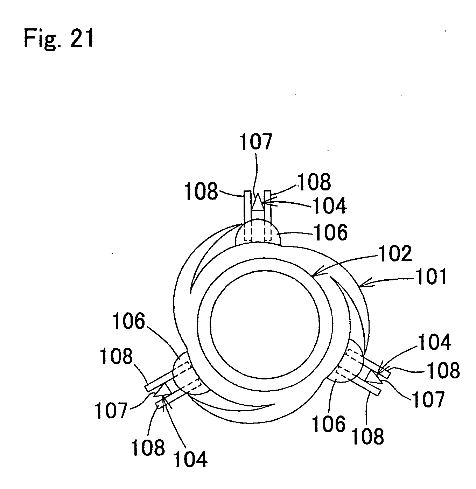

A fast-switching type dilatation catheter for use in coronary arteries having a catheter distal section as illustrated in Fig. 11 was fabricated by fixing three stainless steel cutting blades 104 to the tubular portion of an expandable body 101 made from polyamide elastomer having a nominal expansion diameter of 3.0 mm, by means of adhesive 106, plate-shaped protecting members 108 made from polyamide elastomer material having a Shore hardness of 40D being disposed together with each blade, on either side thereof, the protecting members 108 being disposed in parallel to the blade section 107 of each cutting blade 104, and the height of the protecting members 108 in the circumferential direction being substantially equal to the blade edge. During surgery, the expandable body 101 is initially folded over about the circumference of the tubular member 102 passing through the interior, but since there is no need for protection of the blade edge by the expandable body 101, the expandable body 101 is presented in a folded state in the vicinity of the outer circumference of the tubular member. Fig. 21 shows a simplified cross-sectional view, taken along a plane perpendicular to the axial direction, of the central portion of the expandable body according to the present embodiment when folded over. The maximum diameter of the expandable body portion according to the present embodiment was 1.39 mm, and the bending rigidity according to a three-point evaluation was 320 N·mm².

### (Fourth embodiment)

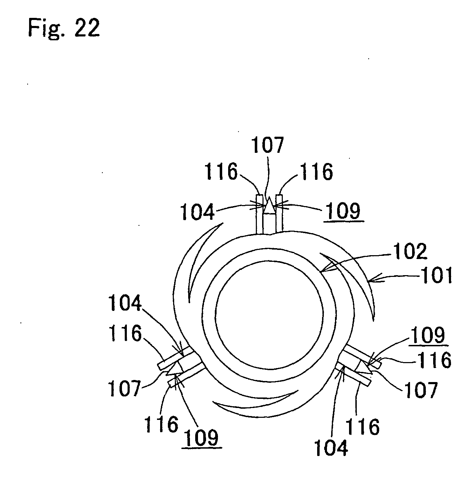

A fast-switching type dilatation catheter for use in coronary arteries having a catheter distal section as illustrated in Fig. 13 was fabricated by forming cutting blade structures 109, each comprising a stainless steel cutting blade 104, plate-shaped protecting members 116 made from a polyamide elastomer material having a Shore hardness of 40D being fixed on either side of the cutting blade 104 and in parallel with the blade section 107 thereof, such that the height of the protecting members 116 in the circumferential direction is substantially the same as that of the blade edge, a distal side supporting section 110 connected to the cutting blade 104, which is made from polyamide elastomer having a Shore hardness of 40D and has a cross-sectional shape of 0.20 mm in the vertical axial direction and 0.50 mm in the horizontal axial direction, and a proximal side supporting section 113 connected to the cutting blade 104, which is made from polyamide elastomer having a Shore hardness of 72D and has a cross-sectional shape of 0.20 mm in the vertical axial direction and 0.50 mm in the horizontal axial direction, and attaching three of such cutting blade structures 109 to tubular members 102, 103 in the vicinity of an expandable body 101 made of polyamide elastomer and having a normal expansion diameter 3.0 mm. The installation of each cutting blade structure 109 is achieved at the distal side by forming the outermost layer of the tubular member 102 from polyamide elastomer, and fusing and fixing the distal side supporting section 110 to the tip section. At the proximal side, the proximal side supporting section 113 is fixed to the tubular member 103 in such a manner that a tension in the axial direction is generated in the cutting blade structure 109. During surgery, the expandable body 101 is initially folded over about the circumference of the tubular member 102 passing through the inside, and the cutting blade structures 109 are presented in a position close to the outer perimeter of the folded over expandable body. Fig. 22 shows a simplified cross-sectional view, taken along a plane perpendicular to the axial direction, of the central portion of an expandable body 101 according to the present embodiment when the expandable body 101 is folded over. The maximum diameter of the expandable body portion according to the present embodiment was 1.37 mm, and the bending rigidity according to a three-point evaluation was 71 N·mm².

### (Comparative example 2)

Comparative example 2 is similar to comparative example 1 illustrated in Fig. 9, being a commercial dilatation catheter comprising three atheroma cutters 19 (metal blades installed on an expandable body by polyurethane adhesive) installed on the outer face of an expandable body 18 having a nominal expansion diameter of 3.0 mm. The maximum diameter of the expandable body portion in comparative example 2 was 1.52 mm and the bending rigidity according to 383 N·mm².

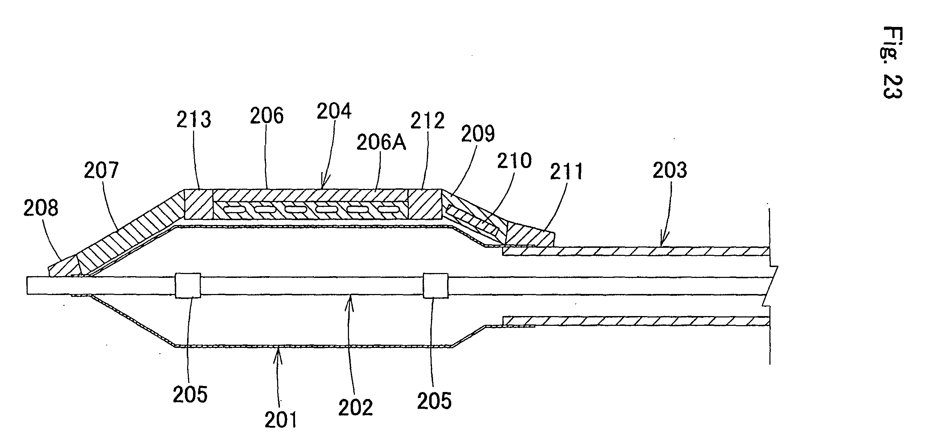

Next, an embodiment of a dilatation catheter relating to a third aspect of the invention is described with reference to Fig. 23 to Fig. 27. The present invention is a dilatation catheter consisting of a plurality of tubular members and an expandable body, characterized in that the dilatation catheter has a structure wherein either end of the expandable body is fixed to the tubular members in the vicinity of the distal side of the catheter, a cutting blade structure consisting of a cutting blade and supporting sections for supporting the cutting blade being installed on the tubular members in the vicinity of the expandable body and at least a portion of the supporting sections being made from a metallic material.

Fig. 23 is a simplified cross-sectional view showing an example in the vicinity of an expandable body, being the distal end of a catheter constituted by an expandable body 201 of a dilatation catheter relating to the third aspect of the invention, a tubular member 202 having a lumen for passing a guide wire, a tubular member 203 for forming the outer face of the catheter and having a lumen for introducing pressure to the expandable body, and a cutting blade structure 204. In Fig. 23, the tubular member 202 having a lumen for passing the guide wire is disposed passing through the inside of the expandable body 201, and is formed into a tip section by being fixed in a concentric fashion with the expandable body at the foremost end of the catheter. The expandable body 201 is connected to the tubular member 203 forming the outer face of the catheter, at the other end thereof. X-ray shielding rings 205 may be fixed to the tubular member 202. The cutting blade structure 204 is constituted by a cutting blade 206, a distal side supporting section 207 and a proximal side supporting section 209, the distal side supporting section 207 being fixed to the tip section formed by the tubular member 202 at a connecting section 208 and to the cutting blade 206 at a connecting section 213, and the proximal side supporting section 209 being fixed to a tubular member 203 at a connecting section 211 and the cutting blade 206 at a connecting section 212.

In Fig. 23, the proximal side supporting section 209 is constituted by a metallic material 210 on the inner side and by resin material on the outer side. There are no particular restrictions on the positioning of the metallic material 210, and it should be provided in at least one portion of the supporting section 207, 209, in such a manner that supporting sections 207, 209 are obtained which are strong with respect to twisting forces, in that they display sufficient angular stability for causing incision when pressed against the object to be incised when the expandable body 201 is expanded, and as illustrated in Fig. 23, desirably, the metallic material 210 is buried in resin. Unless at least one of the portions of the supporting sections 207, 209 are made from a metallic material 210, the cutting blade structure 204 will be liable to twisting and there may be cases where it does not display sufficient incising capability. The cutting blade structure 204 may be fixed to the tubular members 202, 203 at only one end of the expandable body 201, but desirably, the cutting blade structure 204 is attached at either end thereof, as illustrated in Fig. 23, in order that the cutting blade structure 204 is held in a stable fashion. Furthermore, in Fig. 23, only the supporting section 209 on the proximal side is made from a metallic material 210, but it is also possible for the supporting section 207 on the distal side to be made from a metallic material. However, in many cases, a catheter is required to have greater flexibility at the distal side, and especially at the furthest tip thereof, and if sufficient support of the cutting blade structure 204 can be obtained by means of the proximal side supporting section 209 comprising a metallic material 210, then it may be desirable that metallic material is not used as the material for the supporting section 207 on the distal side.

Moreover, when installing the cutting blade structure 204 on a catheter, it is desirable that the cutting blade structure 204 be fixed in such a manner that a tension is generated therein, in the axial direction of the catheter, since this helps to align the cutting blade structure 204 with the catheter axis thereby lowering the profile thereof, as well as helping to position the cutting blade structure 204 in alignment with the expandable body 201 when the expandable body is caused to expand. Here, it is desirable that the distal side supporting section 207 be made from a resin material having a certain degree of flexibility, since this allows the distal side supporting section 207 to perform elastic deformation more readily, and hence it becomes easier to install the cutting blade structure 204 whilst generating a tensile force therein, in the axial direction of the catheter.

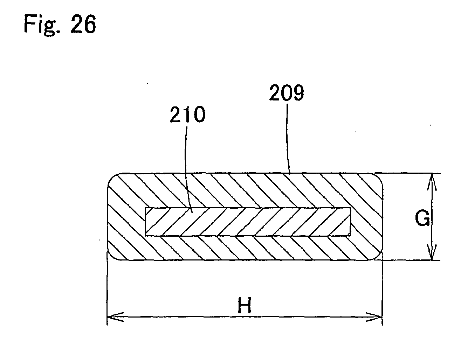

With regard to the minimum thickness of the metallic material 210, a range of 0.03 mm - 0.40 mm is desirable, and a range of 0.07 mm - 0.15 mm is even more desirable, since if the material is too thick, then the external diameter of the catheter as a whole will become too large, whereas if it is too small, then the metallic material will not be able to provide sufficient strength with respect to twisting. As regards the shape of the supporting sections 207, 209, from the viewpoint of being able to provide sufficient strength against twisting in the cutting blade structure 204, and being able to reduce the outer diameter of the catheter as far as possible, it is desirable that the horizontal dimension H is comparatively large with respect to the vertical direction G in the cross-sectional shape of the supporting section 209 perpendicular to the axis of the catheter, as illustrated in Fig. 26, in other words, it is desirable to adopt a flat shape which is elongated in the direction of the circumference of the expandable body 201 (direction of the tangent thereto). There is no particular restriction on the material of the metallic material 210, but stainless steel or superelastic alloys are desirable, due to their excellent balance between rigidity and elasticity, and their extensive use in medical equipment.

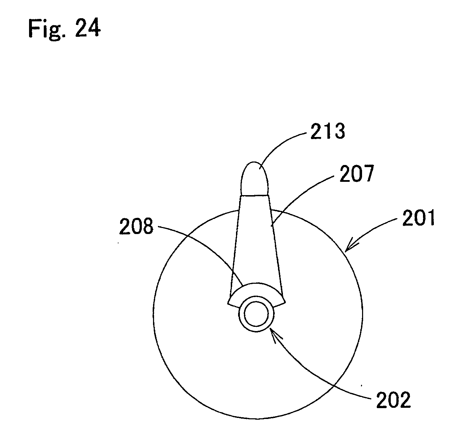

Fig. 24 is a simplified front view of Fig. 23, as viewed from the distal side in the axial direction of the dilatation catheter. In Fig. 24, the tubular member 202 having a lumen for passing a guide wire is formed into a tip section by being fixed in a concentric fashion with the expandable body 201 at the foremost end of the catheter, and the supporting section 207 at the distal side of the cutting blade structure 204 is fixed to the tip section formed by the tubular member 202 at the connecting section 208. Moreover, the cutting blade 206 in the cutting blade structure 204 is desirably positioned in the expandable body portion, as illustrated in Fig. 23, from the object of cutting open the affected region during dilatation, and even more desirably, it is positioned in the central tubular portion of the expandable body 201, since this allows the cutting blade structure 204 to be held readily in a stable fashion. In this case, X-ray shielding rings 205, 205 are desirably fixed to the tubular member 202 so as to mark the end positions of the cutting blade 206.

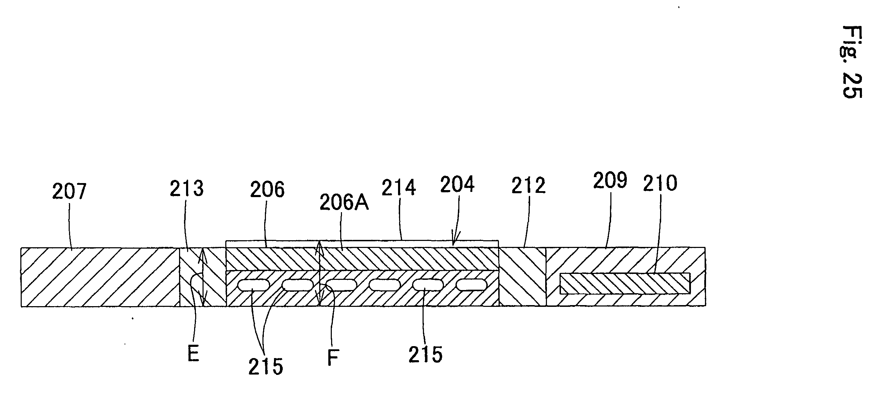

Fig. 25 is an illustrative diagram of a cutting blade structure 204 relating to the third aspect of the invention. A resin cutting blade protecting member 214 is disposed in alignment with the side face of the blade section 206A of the cutting blade 206. The difference between the height E of the supporting sections 207, 209 proximate to the cutting blade 206 in the direction perpendicular to the catheter axis and the height F of the cutting blade 206 in the direction perpendicular to the catheter axis should be small, in order to minimize the amount by which the blade catches on the internal body passages and other equipment used, and a height differential of 0.15 mm or less is desirable, and a height differential of 0.10 mm or less is even more desirable. In this case, the height of F is the height of the protecting member 214. Moreover, the maximum value of the height of the cutting blade structure 204 in the direction perpendicular to the catheter axis should desirably be 0.50 mm or less, and more desirably, 0.35 mm or less, with a view to reducing the outer diameter of the catheter and increasing the flexibility thereof. There is no particular restriction on the number of cutting blade structures 204 installed, and a plurality of cutting blade structures 204 may be installed. When disposing a plurality of cutting blade structures 204, it is desirable that the respective cutting blade structures 204 be installed in such a manner that they are disposed at equidistant intervals about the circumference of the expandable body 201, when the expandable body 201 is caused to expand. Although the use of resin as the material for the cutting blades 206 of the cutting blade structure 204 is possible for achieving the objects, in some cases, the use of metal is more desirable, since it allows the blade section 206A to be adjusted to high sharpness and a desired hardness, and for the metal, stainless steel is desirable, and especially, SUS 400 series steel, and more particularly, SUS 440 steel, is even more desirable. Furthermore, although there are no particular restrictions on the shape of the cutting blade 206, there are cases where it is desirable to provide a plurality of holes 215 for fixing the protecting member 214, on the side face thereof, as illustrated in Fig. 25.

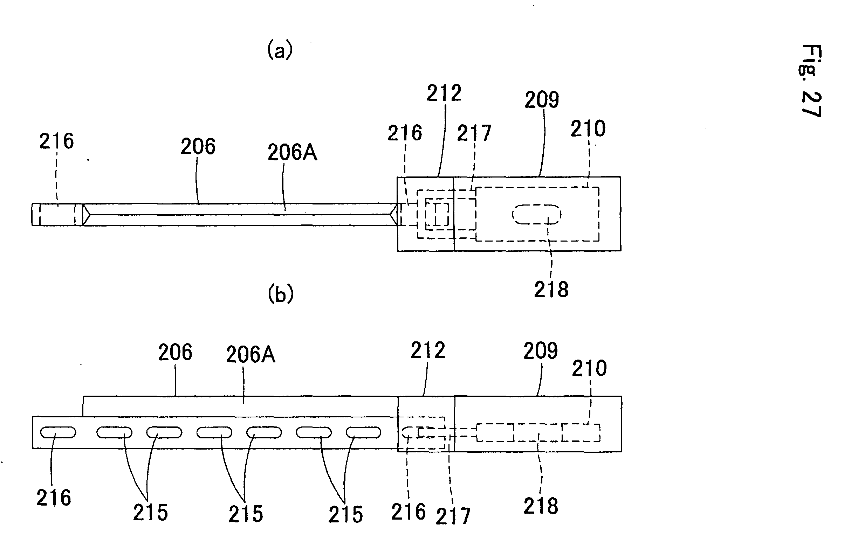

Furthermore, as illustrated in Fig. 27, in some cases, it is desirable to provide holes 216 in the ends portions of the blade member 206 where it connects with the supporting sections 207, 209, in order to strengthen the connection between the cutting blade 206 and the supporting sections 207, 209, and the holes 216 may be formed passing through the metallic material 210, in order to enhance the connection strength. In this example, holes 216 are provided in the end portions of the cutting blade 206, and a ring-shaped portion 217 of the metallic material 210 is disposed so as to pass through the aforementioned hole, the perimeter thereof being covered with resin 218. Moreover, rather than being limited to the example, it is also possible to connect the blade member 206 and metallic material 210. When the metallic material 210 is disposed in a state wherein it is buried in resin, then in some cases it is desirable that the connection strength is increased by forming a hole 218 in the metallic material 210.

### (Embodiments of the third aspect of the invention)

Below, more specific embodiments and comparative examples relating to the third aspect of the invention are described in detail, but the following embodiments do not limit the scope of the present invention in any way.

### (Fifth embodiment)

A cutting blade 206 having a height in the circumferential direction of 0.35 mm was fabricated by fixing plate-shaped protecting members 214 made from a polyamide elastomer material having Shore hardness of 25D to either side of a stainless steel blade section 206A, in a parallel fashion with respect to the blade section and in such a manner that the height of the protecting members 214 in the circumferential direction is substantially the same as the blade edge. A cutting blade structure 209 was formed comprising a cutting blade 206 as described above and, connected to same, a distal side supporting section 207 made from a polyamide elastomer of Shore hardness 25D, having a cross-sectional shape of 0.35 mm in the vertical axial direction and 0.45 mm in the horizontal axial direction, and having a maximum value in the vertical axial direction of 0.35 mm in the supporting section 207 which connects to the cutting blade 206, and a proximal side supporting section 209 comprising an outer layer made from a polyamide elastomer having a Shore hardness of 72D, and a SUS304 stainless steel plate-shaped material (metallic material 210) having a minimum thickness of 0.07 mm disposed on the inner layer thereof, and having a cross-sectional shape of 0.35 mm in the vertical axial direction and 0.45 mm in the horizontal axial direction, the maximum value in the vertical axial direction being 0.35 mm in the supporting section 209 which connects to the cutting blade 206. Three of the aforementioned cutting blade structures 204 were installed on tubular members 202, 203 in the vicinity of an expandable body 201 made from polyamide elastomer having a nominal expansion diameter of 3.0 mm, to fabricate a fast-switching type dilatation catheter for use in coronary arteries having a catheter distal section such as that illustrated in Fig. 23. The installation of the cutting blade structure 204 is achieved by fused and fixing either end thereof to the polyamide elastomer forming the outermost layers of the tubular members 202, 203. During surgery, the expandable body 201 is initially folded over about the circumference of the tubular member 202 passing through the interior thereof, and the cutting blade structures 204 are presented in a position in the vicinity of the outer circumference of the folded over expandable body. After the expandable body has been caused to expand, the expandable body 201 is located in a wing shape between the cutting blade structures 204.

### (Sixth embodiment)

A cutting blade 206 having a height in the circumferential direction of 0.35 mm was fabricated by fixing plate-shaped protecting members 214 made from a polyamide elastomer material having Shore hardness of 25D to either side of a stainless steel blade section 206A, in a parallel fashion with respect to the blade section and in such a manner that the height of the protecting members in the circumferential direction is substantially the same as the blade edge. A cutting blade structure 209 was formed comprising a cutting blade 206 as described above and, connected to same, a distal side supporting section 207 made from a polyamide elastomer of Shore hardness 25D, having a cross-sectional shape of 0.35 mm in the vertical axial direction and 0.45 mm in the horizontal axial direction, and having a maximum value in the vertical axial direction of 0.35 mm in the supporting section 207 which connects to the cutting blade 206, and a proximal side supporting section 209 comprising an outer layer made from a polyamide elastomer having a Shore hardness of 72D, and a SUS304 stainless steel plate-shaped material (metallic material 210) having a minimum thickness of 0.40 mm disposed on the inner layer thereof, and having a cross-sectional shape of 0.50 mm in the vertical axial direction and 0.50 mm in the horizontal axial direction, the maximum value in the vertical axial direction being 0.50 mm in the supporting section 209 which connects to the cutting blade 206. Three of the aforementioned cutting blade structures 204 were installed on tubular members 202, 203 in the vicinity of an expandable body 201 made from polyamide elastomer having a nominal expansion diameter of 3.0 mm, to fabricate a fast-switching type dilatation catheter for use in coronary arteries having a catheter distal section such as that illustrated in Fig. 23. The installation of the cutting blade structure 204 is achieved by fused and fixing either end thereof to the polyamide elastomer forming the outermost layers of the tubular members 202, 203. During surgery, the expandable body 201 is initially folded over about the circumference of the tubular member 202 passing through the interior thereof, and the cutting blade structures 204 are presented in a position in the vicinity of the outer circumference of the folded over expandable body. After the expandable body has been caused to expand, the expandable body 201 is located in a wing shape between the cutting blade structures 204.

### (Seventh embodiment)

A cutting blade 206 having a height in the circumferential direction of 0.35 mm was fabricated by fixing plate-shaped protecting members 214 made from a polyamide elastomer material having Shore hardness of 25D to either side of a stainless steel blade section 206A, in a parallel fashion with respect to the blade section and in such a manner that the height of the protecting members 214 in the circumferential direction is substantially the same as the blade edge. A cutting blade structure 209 was formed comprising a cutting blade 206 as described above and, connected to same, a distal side supporting section 207 made from a polyamide elastomer of Shore hardness 25D, having a cross-sectional shape of 0.25 mm in the vertical axial direction and 0.40 mm in the horizontal axial direction, and having a maximum value in the vertical axial direction of 0.30 mm in the supporting section 207 which connects to the cutting blade 206, and a proximal side supporting section 209 comprising an outer layer made from a polyamide elastomer having a Shore hardness of 72D, a SUS304 stainless steel plate-shaped material (metallic material 210) having a minimum thickness of 0.03 mm disposed on the inner layer thereof, and an SUS304 stainless steel rod-shaped material (ring-shaped member 117) having an outer diameter of 0.09 mm passing through an end hole 216 in the cutting blade 206 and being welded to the stainless steel material (metallic material 210), and having a cross-sectional shape of 0.25 mm in the vertical axial direction and 0.40 mm in the horizontal axial direction, the maximum value in the vertical axial direction being 0.30 mm in the supporting section 209 which connects to the cutting blade 206. Three of the aforementioned cutting blade structures 204 were installed on tubular members 202, 203 in the vicinity of an expandable body 201 made from polyamide elastomer having a nominal expansion diameter of 3.0 mm, to fabricate a fast-switching type dilatation catheter for use in coronary arteries having a catheter distal section such as that illustrated in Fig. 23. The installation of the cutting blade structure 204 is achieved by fused and fixing either end thereof to the polyamide elastomer forming the outermost layers of the tubular members 202, 203. During surgery, the expandable body 201 is initially folded over about the circumference of the tubular member 202 passing through the interior thereof, and the cutting blade structures 204 are presented in a position in the vicinity of the outer circumference of the folded over expandable body. After the expandable body has been caused to expand, the expandable body 201 is located in a wing shape between the cutting blade structures 204.

### (Comparative example 3)

A cutting blade 206 having a height in the circumferential direction of 0.35 mm was fabricated by fixing plate-shaped protecting members 214 made from a polyamide elastomer material having Shore hardness of 25D to either side of a stainless steel blade section 206A, in a parallel fashion with respect to the blade section and in such a manner that the height of the protecting members 214 in the circumferential direction is substantially the same as the blade edge. A cutting blade structure 209 was formed comprising a cutting blade 206 as described above and, connected to same, a distal side supporting section 207 made from a polyamide elastomer of Shore hardness 25D, having a cross-sectional shape of 0.15 mm in the vertical axial direction and 0.30 mm in the horizontal axial direction, and having a maximum value in the vertical axial direction of 0.15 mm in the supporting section 207 which connects to the cutting blade 206, and a proximal side supporting section 209 comprising an outer layer made from a polyamide elastomer having a Shore hardness of 72D, and having a cross-sectional shape of 0.15 mm in the vertical axial direction and 0.40 mm in the horizontal axial direction, the maximum value in the vertical axial direction being 0.15 mm in the supporting section 209 which connects to the cutting blade 206. Three of the aforementioned cutting blade structures 204 were installed on tubular members 202, 203 in the vicinity of an expandable body 201 made from polyamide elastomer having a nominal expansion diameter of 3.0 mm, to fabricate a fast-switching type dilatation catheter for use in coronary arteries having a catheter distal section such as that illustrated in Fig. 23. The installation of the cutting blade structure 204 is achieved by fused and fixing either end thereof to the polyamide elastomer forming the outermost layers of the tubular members 202, 203. During surgery, the expandable body 201 is initially folded over about the circumference of the tubular member 202 passing through the interior thereof, and the cutting blade structures 204 are presented in a position in the vicinity of the outer circumference of the folded over expandable body. After the expandable body has been caused to expand, the expandable body 201 is located in a wing shape between the cutting blade structures 204.

### (Comparative example 4)

Comparative example 4 is similar to comparative example 1 illustrated in Fig. 9, being a commercial dilatation catheter constituted by three atheroma cutters 19 (metal blades attached to an expandable body by polyurethane adhesive) which are installed in the outer face of the of an expandable body 18. In the case of comparative example 4, the base portion of the blade is fixed by adhesive, and the exposed height of the blade in the circumferential direction is approximately 0.18 mm.

### (Evaluation)

The fifth, sixth and seventh embodiments and comparative example 3 were caused to expand in a constricted region of imitation constricted internal body passages fabricated from silicon resin having a variety of hardnesses. When comparative example 3 was caused to expand in an imitation constricted internal body passage of high hardness, twisting occurred in the cutting blade structure, and incisions could not be inserted properly in the constricted region. In the case of each of the fifth, sixth and seventh embodiments of the present invention, good incisions were performed. Moreover, an experiment was conducted wherein the devices were caused to expand in, and to the distal side of, a metallic stent section held in the of left anterior descending coronary artery of a pig's heart, the resistance when removing same was measured using a load meter and in the event of any catching of the devices, the corresponding site was confirmed by X-ray contrast imaging. In the fifth, sixth and seventh embodiments, there was no catching between the metallic stent section and other portions of the blood vessel, and the catheter and cutting blade structure inside a guiding catheter (outer diameter 6F (2 mm), internal diameter 0.070 inch (1.78 mm)), and the resistance value was approximately 0.3N. In the case of comparative example 3, there was some catching between the stent section and the front tip of the guiding catheter, and in some cases, the resistance value in this situation exceeded approximately 1.0 N. In the case of comparative example 4, there was some catching between the stent section and the front tip of the guiding catheter, and in some cases, the resistant value exceeded approximately 1.0 N. Furthermore, in some cases, fissures was caused in the front tip of the guiding catheter.

### Industrial Applicability

As described above, the medical dilatation catheter according to the first aspect of the invention is a dilatation catheter for performing dilatation of an internal body passage whilst inserting incisions into the region being dilated, but compared to the prior art, the expandable body portion thereof is fine and has excellent flexibility, and hence this dilatation catheter provides excellent operability, particularly in terms of the ease of introducing same into constricted affected regions, and curved affected regions. Moreover, the expandability characteristics (compliance) of the expandable body can be controlled freely.

Furthermore, the medical dilatation catheter according to the second aspect of the invention is a dilatation catheter for performing dilatation whilst inserting incisions into the region being dilated, which provides excellent safety by increasing the protection of the cutting blades used to insert incisions. Moreover, it also provides a dilatation catheter for performing dilatation whilst inserting incisions in the region being dilated, which has improved flexibility in the expandable body portion.

Furthermore, the medical dilatation catheter according to the third aspect of the invention is a dilatation catheter for performing dilatation whilst inserting incisions in the region being dilated, which has excellent incising capability and excellent safety.

## Claims

1. A dilatation catheter comprising a plurality of tubular members and an expandable body, wherein a cutting blade structure is installed on the tubular members in the vicinity of the expandable body.

2. The dilatation catheter comprising a plurality of tubular members and a expandable body according to claim 1, wherein the dilation catheter has a structure in which the opposite ends of the expandable body are fixed to the tubular members in the vicinity of the distal end of the catheter, a cutting blade structure is installed on the tubular members in the vicinity of the expandable body.

3. The dilatation catheter according to claim 1 or claim 2, wherein the cutting blade structure is installed on said tubular members at either end of said expandable body, in such a manner that the cutting blade of said cutting blade structure is positioned on the surface of the expandable body.

4. The dilatation catheter according to any one of claims 1 to 3, wherein said cutting blade structure is constituted by a cutting blade and supporting sections for supporting the cutting blade and is installed in such a manner that the edge of said cutting blade is oriented in a perpendicular direction to the surface of the expanded expandable body, when the expandable body is caused to expand, and the cross-sectional shape of said supporting section in the direction perpendicular to the axis of the catheter is a flat shape which is elongated in the direction of the tangent to the circumference of the expandable body.

5. The dilatation catheter according to any one of claims 1 to 4, wherein said cutting blade structure is fixed in such a manner that a tension is generated in the axial direction thereof.

6. The dilatation catheter according to any one of claims 1 to 5, wherein said cutting blade structure is connected to a coil-shaped elastic member and is fixed in such a manner that a tension is generated in the cutting blade structure in the axial direction thereof, by means of the stress occurring in the coil-shaped elastic member.

7. The dilatation catheter according to any one of claims 1 to 6, wherein the material of the blade section of the cutting blade of said cutting blade structure is metal.

8. The dilatation catheter according to claim 7, wherein the material of the blade section of the cutting blade of said cutting blade structure is stainless steel.

9. The dilatation catheter according to any one of claims 1 to 8, wherein said cutting blade structure is constituted by a cutting blade and supporting sections for supporting the cutting blade, the material of said supporting sections being resin.

10. The dilatation catheter according to claim 9, wherein said cutting blade structure is constituted by a cutting blade and supporting sections for supporting the cutting blade, and the material of said supporting sections is a resin which can be fused with said tubular members of the catheter.

11. The dilatation catheter according to claim 10, wherein at least a part of the portion of said tubular members where they are fixed with said cutting blade structure is formed into a multi-layer structure of two or more layers.

12. The dilatation catheter according to any one of claims 9 to 11, wherein the material of the supporting section on the distal side of said cutting blade structure is set to a lower hardness than the material of the proximal side supporting section.

13. The dilatation catheter according to any one of claims 1 to 11, wherein the expandability characteristics (compliance) of the expandable body is 0.80% per atmosphere (0.78%/MPa), or above.

14. A dilatation catheter comprising a plurality of tubular members, an expandable body, and a cutting blade disposed in the vicinity of the expandable body, wherein the dilatation catheter further comprises protecting members for protecting the blade portion of the cutting blade.

15. The dilatation catheter comprising a plurality of tubular members, an expandable body, and a cutting blade disposed in the vicinity of the expandable body according to claim 14, wherein the dilatation catheter further comprises protecting members having the functions of protecting the blade section of the cutting blade and exposing the blade section of the cutting blade when the cutting blade is pressed in the outward radial direction of the expandable body.

16. The dilatation catheter according to claim 14 or claim 15, wherein said protecting members for protecting the blade section of said cutting blade are disposed in parallel with the blade section of the cutting blade on at least one side of the cutting blade.

17. The dilatation catheter according to claim 16, wherein said protecting members for protecting the blade section of said cutting blade have a flat plate-shaped structure at least in a part of the portion thereof disposed in parallel with the blade section of the cutting blade, and the flat plate-shaped portion has approximately the same height as the blade section of the cutting blade.

18. The dilatation catheter according to any one of claims 14 to 17, wherein said protecting members for protecting the blade section of said cutting blade are made of resin.

19. The dilatation catheter according to any one of claims 14 to 18, wherein said cutting blade is fixed to said expandable body.

20. The dilatation catheter according to any one of claims 14 to 18, wherein said cutting blade structure having said cutting blade is attached to said tubular members in the vicinity of the expandable body.

21. The dilatation catheter according to claim 20, wherein the width of the base portion of the cutting blade in said cutting blade structure is at least 1/2 or more of the height of the cutting blade.

22. A dilatation catheter comprising a plurality of tubular members and an expandable body, wherein the dilatation catheter has a structure in which the opposite ends of the expandable body are fixed to the tubular members in the vicinity of the distal end of the catheter, a cutting blade structure constituted by a cutting blade and supporting sections for supporting the cutting blades is attached to the tubular members in the vicinity of the expandable body by means of the supporting sections, and at least a part of the supporting sections is made from a metallic material.

23. The dilatation catheter according to claim 22, wherein the minimum thickness of said metallic material is in the range of 0.03 mm to 0.40 mm.

24. The dilatation catheter according to claim 22 or claim 23, wherein the cross-sectional shape of said supporting sections in a direction perpendicular to the axis of the catheter is a flat shape which is elongated in the direction of the tangent to the circumference of said expandable body.

25. The dilatation catheter according to any one of claims 22 to 24, wherein said metallic material is stainless steel or a superelastic alloy.

26. The dilatation catheter according to any one of claims 22 to 25, wherein said metallic material is disposed in a state where the metallic material is buried inside a resin.

27. The dilatation catheter according to any one of claims 22 to 26, wherein said metallic material is connected to said cutting blade, or alternatively, is disposed such that it passes through a hole portion provided in the cutting blade.

28. The dilatation catheter according to any one of claims 22 to 27, wherein supporting sections of said cutting blade structure are attached to the tubular members on either side of the expandable body and in the vicinity thereof, and at least a part of the supporting section on the proximal side only is made from a metallic material.

29. A dilatation catheter comprising a plurality of tubular members and an expandable body, wherein the dilatation catheter has a structure in which the opposite ends of an expandable body are fixed to the tubular members in the vicinity of the distal end of the catheter, a cutting blade structure constituted by a cutting blade and supporting sections for supporting the cutting blade is attached to the tubular members in the vicinity of the expandable body, by means of the supporting sections, and the difference between the height of the supporting sections adjacent to the cutting blade, in a perpendicular direction to the axis of the catheter, and the height of the cutting blade in a perpendicular direction to the axis of the catheter, is 0.15 mm or less.

30. A dilatation catheter comprising a plurality of tubular members and an expandable body, wherein the dilatation catheter has a structure in which the opposite ends of the expandable body are fixed to the tubular member in the vicinity of the distal end of the catheter, a cutting blade structure constituted by a cutting blade and supporting sections for supporting the cutting blade is attached to the tubular members in the vicinity of the expandable body, by means of the supporting sections, and the maximum value of the height of the cutting blade structure in the perpendicular direction to the axis of the catheter is 0.50 mm or less.
